# EUROPEAN PATENT SPECIFICATION

(11) **EP 3 169 321 B1**
(45) Date of publication and mention of the grant of the patent: **15.07.2020**
(21) Application number: 15822404.8
(22) Date of filing: 17.07.2015
(51) Int. Cl.: A61K 31/216, A61K 31/353, A61K 35/644, A61K 47/40, A61P 35/00, A61K 9/00, A61K 31/192, A61K 31/724

(54) **PROPOLIS AND EXTRACTS THEREOF FOR THE TREATMENT OF SKIN CANCERS AND IMPROVEMENT OF SKIN HEALTH**
PROPOLIS UND EXTRAKTE DARAUS ZUR BEHANDLUNG VON HAUTKARZINOMEN UND VERBESSERUNG DER HAUTGESUNDHEIT
PROPOLIS ET EXTRAITS DE PROPOLIS POUR TRAITER LES CANCERS DE LA PEAU ET AMÉLIORER LA SANTÉ DE LA PEAU

(30) Priority: 18.07.2014 NZ 62718914
(43) Date of publication of application: 24.05.2017
(73) Proprietor: Manuka Health New Zealand Limited, Mount Wellington, Auckland 1072 (NZ)
(72) Inventor: SUDDES, Amanda Jan, Waitakere, Auckland 0782 (NZ); CATCHPOLE, Owen John, Lower Hutt 5040 (NZ)
(74) Representative: Mewburn Ellis LLP
(86) International application number: PCT/NZ2015/050093
(87) International publication number: WO 2016/010440

(56) References cited:
- WO-A1-2004/050063
- WO-A1-2004/089392
- WO-A1-2011/057686
- WO-A1-2014/163512
- WO-A1-2014/163513
- WO-A2-02/47615
- WO-A2-2013/022740
- CN-A- 1 439 356
- MARIA ANG?LICA EHARA WATANABE ET AL: "Cytotoxic constituents of propolis inducing anticancer effects: a review : Anticancer effects of propolis", JOURNAL OF PHARMACY AND PHARMACOLOGY, vol. 63, no. 11, 27 September 2011 (2011-09-27), pages 1378-1386, XP55386174, LONDON; GB ISSN: 0022-3573, DOI: 10.1111/j.2042-7158.2011.01331.x
- CONEAC, G. ET AL.: 'Propolis extract/beta-cyclodextrin nanoparticles: synthesis, physico- chemical, and multivariate analyses' JOURNAL OF AGROALIMENTARY PROCESSES AND TECHNOLOGIES vol. 14, 2008, pages 58 - 70, XP002658835
- USIA, T. ET AL.: 'Constituents of Chinese Propolis and Their Antiproliferative Activities' JOURNAL OF NATURAL PRODUCTS vol. 65, no. 5, 2002, pages 673 - 676, XP055285565
- LI, F. ET AL.: 'Cytotoxicity of Constituents from Mexican Propolis Against a Panel of Six Different Cancer Cell Lines' NATURAL PRODUCT COMMUNICATIONS vol. 5, no. 10, 2010, pages 1601 - 1606, XP055291824
- WATANABE, M.A.E. ET AL.: 'Cytotoxic constituents of propolis inducing anticancer effects: a review' JOURNAL OF PHARMACY AND PHARMACOLOGY vol. 63, 2011, pages 1378 - 1386, XP055386174

## Description

### FIELD OF INVENTION

This invention relates to compositions for use in skin care and in the treatment and prevention of skin cancers. In particular, this invention relates to anti-skin cancer compositions containing propolis and cyclodextrin, including anti-skin cancer compositions comprising one or more propolis extracts, and/or anti-skin cancer compositions containing one or more compounds derived from propolis, including anti-skin cancer compositions comprising one or more propolis extracts enriched in one or more of these compounds. Particularly contemplated is the use of such compositions in the treatment or prevention of skin disorders, and skin cancers, such as basal cell carcinomas, squamous cell carcinomas, and melanomas.

### BACKGROUND OF THE INVENTION

Crude propolis is a resinous substance produced by bees from the resin collected from botanical sources, such as buds, sap, and leaf exudates which is admixed with beeswax. The colour of crude propolis can vary from yellow, through browns to almost black depending on the botanical source. Beeswax is normally separated from the propolis by extraction using ethanol, in which the wax is insoluble but the resinous compounds are highly soluble. There are a number of types of propolis, which are based on the botanical sources of resin compounds, and the geographical region. The most well-known types of propolis are "European" propolis, where the resin compounds are obtained by bees mostly from leaf and bud exudates of poplars, and to a lesser extent birches and willows; and "Brazilian Green" propolis, which is mainly obtained by bees from leaf exudates of the tree *Baccharis dracanculifolia.* Propolis produced in New Zealand can be categorized as "European" as its composition broadly matches other European propolis (Markham et al, 1996. HPLC and GC-MS identification of the major organic constituents in New Zealand propolis. Phytochemistry, 42(1): 205-211). Crude propolis and extracts derived from propolis have been reported to have antibacterial, antifungal, and antiviral activities. Its use in the treatment of several types of cancer, in particular breast cancer, has also been investigated.

Identification and verification of the anti-cancer constituent(s) present in propolis resin has been challenging because of the complex and multicomponent nature of the resin. In Brazilian Green propolis, the anti-cancer activity is mainly attributed to artepillin C, while in European propolis resin the anti-cancer activity is mainly attributed to caffeic acid phenethyl ester (CAPE), a dihydroxy cinnamic acid ester; and chrysin, an aglycone flavonoid, see for example, Sawicka et al (2012) "The anticancer activity of propolis", Folia Histochemica et Cytobiologica, 50 (1), 25-37.

Skin cancers are among the most prevalent cancers in the world, with more than 3 million diagnosed cases annually in the USA alone. Skin cancer rates are particularly high in Australia and New Zealand, with incidence being as much as 4 times greater than in the USA. While surgery can be effective, early detection is critical to positive surgical outcomes. Other therapies are largely directed at low risk disease, as the efficacy of current chemotherapies and radiotherapies in treating primary tumours, particularly of malignant melanomas, or metastases outside the lymph nodes, is debated.

Accordingly, there is a need for anti-skin cancer compositions, including those suitable for use in the treatment or prevention of basal cell carcinoma, squamous cell carcinoma, and melanoma, and those which are able to support the maintenance of anti-skin cancer activity or augment anti-skin cancer activity.

It is an object of the present invention to provide anti-skin cancer compositions for use in the treatment or prevention of skin cancer, including basal cell carcinoma, squamous cell carcinoma, and melanoma, and/or to provide compositions for improving skin health, or to at least provide the public with a useful choice.

Watanabe et al., (2011) "Cytotoxic constituents of propolis inducing anticancer effects: a review" JPP, 63: 1378-1386, concerns the effect of propolis on experimental carcinogenesis models and mechanisms of anti-tumorigenesis.

### SUMMARY OF THE INVENTION

The invention provides a composition comprising, consisting essentially of, or consisting of propolis and cyclodextrin for use in treating or preventing skin cancer, wherein at least some of the cyclodextrin present in the composition is present as an inclusion complex with one or more compounds present in the propolis, and wherein the composition is formulated for topical administration.

In some embodiments the composition finds use in treating or preventing skin cancer wherein the skin cancer is a melanoma, squamous cell carcinoma or a basal cell carcinoma.

In some embodiments the cyclodextrin is alpha-cyclodextrin, beta-cyclodextrin, gamma-cyclodextrin or a chemically-modified cyclodextrin, or the cyclodextrin is present as a combination of cyclodextrins comprising alpha-cyclodextrin, beta-cyclodextrin, gamma-cyclodextrin, or a combination of any two or more thereof.

In some embodiments the composition comprises from 1.0%wt to 50 %wt propolis or from 1% to 30%wt propolis resin.

In some embodiments the composition comprises two or more of caffeic acid phenylether ester (CAPE), caffeic acid, pinocembrin, benzyl caffeate, benzyl ferulate, cinnamyl caffeate, cinnamyl ferulate, chrysin, galangin, pinostrobin chalcone, and pinobanksin.

In some embodiments the composition comprises pinobanksin-3-acetate.

In some embodiments the composition has:
a) a CAPE concentration of greater than 1 mg/g,
b) a pinocembrin concentration of greater than 10 mg/g,
c) a galangin concentration of greater than 5 mg/g,
d) a chrysin concentration of greater than 5 mg/g,
e) a caffeic acid concentration of greater than 1 mg/g or
f) a pinobanksin concentration of greater than 1 mg/g.

In some embodiments the composition is for use in treating or preventing skin cancer, wherein the skin cancer is a squamous cell carcinoma or a basal cell carcinoma, and wherein the composition comprises a compound of formula (II): 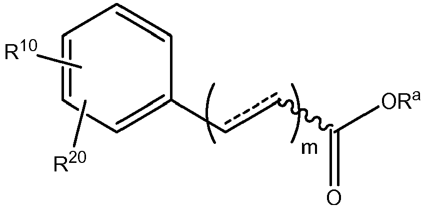 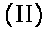
wherein:
   Ra is hydrogen, C2-6alkenyl, arylC1-6alkyl, or arylC2-6alkenyl;
   m is 1 or 2; and
   R10 and R20 are each independently hydrogen, hydroxyl, or C1-6alkoxy; and
   provided that Ra is not arylC1-6alkyl or arylC2-6alkenyl when R10 and R20 are both hydroxyl and m is 1;
wherein
   preferably the C2-6alkenyl is prenyl or isoprenyl,
   preferably the arylC1-6alkyl is benzyl,
   preferably the arylC2-6alkenyl is cinnamyl,
   preferably the C1-6alkoxy is OMe;
wherein at least some of the compound is present as an inclusion complex with cyclodextrin.

In some embodiments of said composition, the compound of formula (II) is a compound of the formula (IIA):

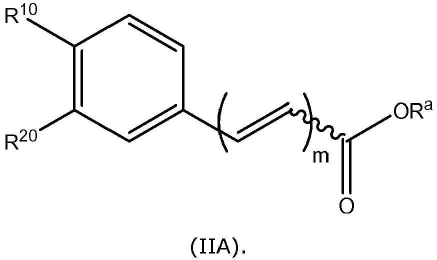

In some embodiments the composition contains the compound of formula (II) or (IIA), wherein R10 and R20 are each hydrogen, R10 and R20 are each hydroxyl, R10 is hydroxyl and R20 is C1-6alkoxy, or R10 is C1-6alkoxy and R20 is hydroxyl.

In some embodiments the composition contains the compound of formula (II) or (IIA), wherein Ra is hydrogen, C2-6alkenyl, or arylC1-6alkyl.

In some embodiments the composition contains the compound of formula (II) or (IIA), wherein m is 1.

In some embodiments the composition contains the compound of formula (II) or (IIA), wherein Ra is C2-6alkenyl or arylC1-6alkyl; and R10 and R20 are each hydroxyl, R10 is hydroxyl and R20 is C1-6alkoxy, or R10 is C1-6alkoxy and R20 is hydroxyl.

In some embodiments the composition contains the compound of formula (II) or (IIA), wherein
m is 2, Ra is hydrogen, and R10 and R20 are each independently hydrogen, hydroxyl, or C1-6alkoxy;
m is 1 or 2, Ra is C2-6alkenyl, and R10 and R20 are each independently hydrogen, hydroxyl, or C1-6alkoxy; or
m is 1 or 2, Ra is arylC1-6alkyl, R20 is hydrogen, hydroxyl, or C1-6alkoxy, and R10 is C1-6alkoxy.

In some embodiments the composition contains the compound of formula (II), wherein the compound is selected from the group consisting of
a) 1,1-dimethylallyl caffeate,
b) 3-methyl-3-butenyl caffeate,
c) 5-phenylpenta-2,4-dienoic acid, and
d) benzyl isoferulate.

In some embodiments the composition is formulated for topical administration to a dermal or epidermal surface of a subject.

In one embodiment, in accordance with the claims, the composition comprises an inclusion complex of cyclodextrin and a compound of formula (IIA) or a compound selected from any one or more of
a) 5-phenylpenta-2,4-dienoic acid,
b) 3-methyl-3-butenyl caffeate,
c) 1,1-dimethylallyl caffeate,
d) pinobanksin-3-acetate, and
e) benzyl isoferulate.

In another embodiment in accordance with the claims, the invention relates to an anti-skin cancer composition comprising at least one compound as defined herein, including a compound of formula (II), or a compound selected from any one or more of
f) 5-phenylpenta-2,4-dienoic acid,
g) 3-methyl-3-butenyl caffeate,
h) 1,1-dimethylallyl caffeate,
i) pinobanksin-3-acetate, and
j) benzyl isoferulate.
For the avoidance of doubt, 5-phenylpenta-2,4-dienoic acid has formula (A), 3-methyl-3-butenyl caffeate has formula (B), 1,1-dimethylallyl caffeate has formula (C), pinobanksin-3-acetate has formula (D), and benzyl isoferulate has formula (E) as shown below.

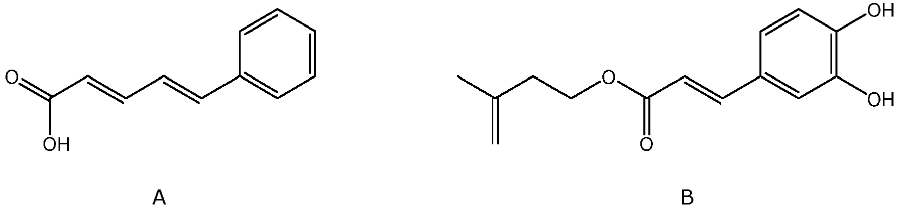

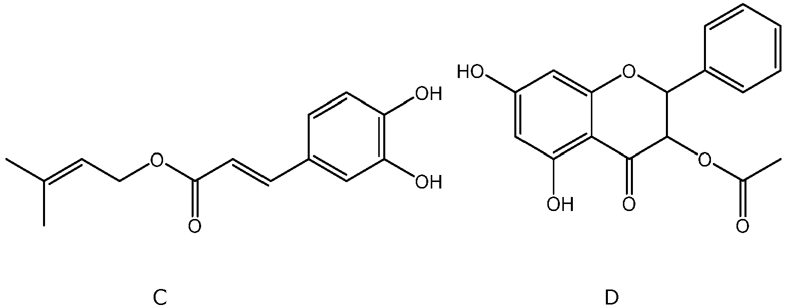

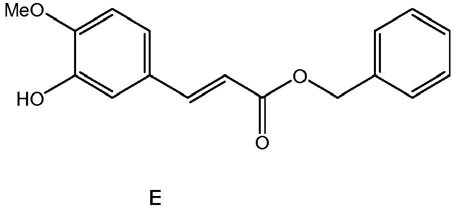

In one embodiment, the invention relates to a composition for use in the treatment or prevention of melanoma, the composition comprising a therapeutically effective amount of a compound selected from any one or more of
a) 5-phenylpenta-2,4-dienoic acid,
b) 3-methyl-3-butenyl caffeate,
c) 1,1-dimethylallyl caffeate,
d) pinobanksin-3-acetate, and
e) benzyl isoferulate.

In a further embodiment, the invention relates to a composition, as defined in the claims, for use in inhibiting skin tumour formation, inhibiting skin tumour growth, inhibiting skin tumour metastasis or treating or preventing skin cancer in a human subject; inducing apoptosis of one or more neoplastic skin cells in a human subject; increasing the responsiveness of a human subject to a skin cancer therapy; increasing the sensitivity of a skin tumour in a human subject to a skin cancer therapy; resensitising one or more skin cancer cells in a human subject that are resistant to treatment; at least partially reversing the resistance of a neoplastic cell in a human subject suffering from skin cancer to a skin cancer therapy; reversing, wholly or in part, the resistance of a skin cancer-burdened human patient to a skin cancer therapy; or re-sensitising one or more tumours of a skin cancer-burdened human patient which are, or are predicted to either be or become, resistant to treatment with a skin cancer therapy to treatment with a skin cancer therapy.

In a further embodiment essentially all of the one or more compounds present in the composition, as defined in the claims, is present as an inclusion complex with cyclodextrin.

In various embodiments, the composition comprises at least about 5 % w/w propolis solids. For example, the composition comprises at least about 7.5% w/w propolis solids, at least about 10 % w/w propolis solids, at least about 12.5% w/w propolis solids, at least about 15 % w/w propolis solids, at least about 17.5% w/w propolis solids, or at least about 20 % w/w propolis solids.

In one embodiment, the cyclodextrin is chemically-modified cyclodextrin, examples of which are described in Stella and He, Cyclodextrins, Toxicologic Pathology, 36, 2008, 30-42.

For example, the cyclodextrin is hydroxypropyl beta-cyclodextrin, or the cyclodextrin is present as a combination of cyclodextrins comprising hydroxypropyl beta-cyclodextrin. For example, the composition comprises an inclusion complex of hydroxypropyl beta-cyclodextrin and one or more compounds present in propolis, for example one or more of the compounds described herein.

In one embodiment, the propolis is present in the anti-skin cancer composition as a propolis extract or fraction.

In one embodiment, the propolis present in the anti-skin cancer composition is free of wax. For example, the propolis has been dewaxed using extraction processes known in the art. Dewaxed propolis is often known as 'propolis resin'.

In one embodiment, the propolis is "European" or "Poplar" propolis. For example, the "European" propolis is at least in part derived from the bud and leaf exudates of one or more species of poplars, birches, larches or willows. In another embodiment, the propolis is "Brazilian Green" propolis. For example, the "Brazilian Green" propolis is at least in part obtained by bees from leaf exudates of the tree *Baccharis dracanculifolia.*

In one embodiment, the composition comprises from about 1.0%wt to about 99 %wt propolis.

In one embodiment the molar ratio of propolis to cyclodextrin in the composition, as defined in the claims, is no greater than about 1:1.

In various embodiments, the one or more compounds of formula (II) is present in the composition at a concentration of greater than about 1mg/g up to about 1000mg/g.

In various embodiments, the propolis of the composition as defined in the claims, for example, the European propolis, comprises any combination of two or more of CAPE, chrysin, galangin, pinocembrin, pinobanksin, benzyl caffeate, benzyl ferulate, cinnamyl caffeate, cinnamyl ferulate, pinostrobin chalcone, and caffeic acid.

In one embodiment, the propolis has a CAPE concentration of greater than about 1mg/g up to about 1000mg/g.

In one embodiment, the propolis has a pinocembrin concentration of greater than about 1mg/gup to about 1000mg/g.

In one embodiment, the propolis has a galangin concentration of greater than about 1mg/g up to about 1000mg/g.

In one embodiment, the propolis has chrysin concentration of greater than about 1mg/g up to about 1000mg/g.

In one embodiment, the propolis has benzyl caffeate concentration of greater than about 1mg/gup to about 1000mg/g.

In one embodiment, the propolis has benzyl ferulate concentration of greater than about 1mg/g up to about 1000mg/g.

In one embodiment, the propolis has cinnamyl caffeate concentration of greater than about 1mg/gup to about 1000mg/g.

In one embodiment, the propolis has cinnamyl ferulate concentration of greater than about 1mg/g up to about 1000mg/g.

In exemplary embodiments, the composition, as defined in the claims, is one to which has been added one or more of CAPE, caffeic acid, pinocembrin, benzyl caffeate, benzyl ferulate, benzyl isoferulate, cinnamyl caffeate, cinnamyl ferulate, pinostrobin chalcone, chrysin, galangin, and pinobanksin.

In various embodiments, the composition, as defined in the claims, comprises or is administered separately, simultaneously or sequentially with at least one additional therapeutic agent, for example the at least one additional therapeutic agent is an anti-tumour agent, for example the anti-tumour agent is selected from an anti-tumour food factor, a chemotherapeutic agent, or an immunotherapeutic agent.

In various embodiments, the skin cancer therapy, the therapeutic agent, or the anti-tumour agent is effective to induce apoptosis, for example, induce apoptosis in one or more skin cancer cells or in one or more neoplastic cells.

In one embodiment, the topical composition, as defined in the claims, comprises one or more penetrants, photo-protectants, UV-protectants, vitamins, moisturizers, oils, hydrophilic or lipophilic gelling agents, hydrophilic or lipophilic active agents, preserving agents, antioxidants, solvents, fragrances, fillers, pigments, odor absorbers or dyestuffs.

In one embodiment, the composition comprises one or more additional anti-skin cancer agents.

In one embodiment, the composition is a pharmaceutical composition.

In various embodiments, the chemotherapeutic agent is selected from the group comprising mitotic inhibitors, such as vinca alkaloids, including vincristine, vinblastine, vinorelbine, vindesine, vinflunine, podophyllotoxin, taxanes, including docetaxel, larotaxel, ortataxel, paclitaxel, and tesetaxel, and epothilones, such as ixabepilone; topoisomerase I inhibitors, such as topotecan, irinotecan, camptothecin, rubitecan, and belotecan, topoisomerase type II inhibitors, including amsacrine, etoposide, etoposide phosphate, and teniposide, anthracyclines, such as aclarubicin, daunorubicin, doxorubicin, epirubicin, idarubicin, amrubicin, pirarubicin, valrubicin, and zorubicin, and anthracenediones, such mitoxantrone and pixantrone; antimetabolites, including dihydrofolate reductase inhibitors, such as aminopterin, methotrexate, pemetrexed, thymidylate synthase inhibitors, such as raltitrexed and pemetrexed, adenosine deaminase inhibitors, including pentostatin, halogenated or ribonucleotide reductase inhibitors, such as cladribine, clofarabine, and fludarabine, thiopurines, including thioguanine and mercaptopurine, thymidylate synthase inhibitors, including fluorouracil, capecitabine, tegafur, carmofur, and floxuridine, DNA polymerase inhibitors, such as cytarabine, ribonucleotide reductase inhibitors, such as gemcitabine, hypomethylating agents, including azacitidine, and decitabine, and ribonucleotide reductase inhibitors, such as hydroxyurea; cell-cycle nonspecific antineoplastic agents, including alkylating agents such as nitrogen mustards, including mechlorethamine, cyclophosphamide, ifosfamide, trofosfamide, chlorambucil, melphalan, prednimustine, bendamustine, uramustine, estramustine, nitrosoureas, including carmustine, lomustine, semustine, fotemustine, nimustine, ranimustine, and streptozocin, alkyl sulfonates, including busulfan, mannosulfan, and treosulfan, aziridines, including carboquone, thioTEPA, triaziquone, and triethylenemelamine, alkylating-like agents, including platinum agents such as cisplatin, carboplatin, oxaliplatin, nedaplatin, triplatin tetranitrate, satraplatin, hydrazines, such as procarbazine, triazenes, such as dacarbazine, temozolomide, altretamine, and mitobronitol, and streptomycins, such as actinomycin, bleomycin, daunomycin,mitomycin, and plicamycin; photosensitizers, including aminolevulinic acid, methyl aminolevulinate, efaproxiral, and porphyrin derivatives, such as porfimer sodium, talaporfin, temoporfin, and verteporfin; enzyme inhibitors, including farnesyltransferase inhibitors such as tipifarnib, cyclin-dependent kinase inhibitors, such as alvocidib and seliciclib, proteasome inhibitors, such as bortezomib, phosphodiesterase inhibitors, such as anagrelide, IMP dehydrogenase inhibitors, such as tiazofurine, lipoxygenase inhibitors, such as masoprocol, and PARP inhibitors, such as olaparib; receptor antagonists, such as endothelin receptor antagonists including atrasentan, retinoid X receptor antagonists, such as bexarotene, and testolactone; and other chemotherapeutics, including amsacrine, trabectedin, retinoids such as alitretinoin and tretinoin, arsenic trioxide, asparagine depleters such as asparaginase or pegaspargase, celecoxib, demecolcine, elesclomol, elsamitrucin, etoglucid, and lonidamine.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

### DETAILED DESCRIPTION

The present invention provides compositions comprising propolis and cyclodextrin; and particularly beta cyclodextrin or gamma-cyclodextrin, for example compositions comprising European propolis and beta-cyclodextrin or gamma-cyclodextrin, that have anti-skin cancer efficacy, including enhanced efficacy, and preventative activity. The anti-skin cancer compositions of the invention, as defined in the claims, enhance the activity and physicochemical properties of propolis. The synergistic effect of propolis and cyclodextrin in the compositions of the invention allow for controlled release of propolis following administration to a subject, improved bioavailability, and/or reduced allergenicity.

Furthermore, in one aspect the present invention provides compositions, as defined in the claims, comprising compounds selected from any one or more of 5-phenylpenta-2,4-dienoic acid, 3-methyl-3-butenyl caffeate, 1,1-dimethylallyl caffeate, pinobanksin-3-acetate, and benzyl isoferulate that have anti-skin cancer activity. The anti-cancer compositions of the invention are used to treat or prevent skin cancer, and in some embodiments can be used to enhance the activity and physicochemical properties of propolis or materials with propolis contained.

Accordingly, provided that the anti-skin cancer compositions of the invention are formulated so as to be suitable for administration to a mammalian subject, for example they consist of materials that are safe to the human body, they can be used for manufacturing anti-skin cancer pharmaceutical compositions, as well as lotions, and skin creams.

Furthermore, the anti-skin cancer activity of embodiments of the compositions of the invention, as defined in the claims, may be maintained for a sustained periodsuch that the dosage or frequency of administration of the composition can be reduced, or higher efficacy is provided, or both.

The phrases "anti-skin cancer compositions" or "compositions having anti-skin cancer activity" are used interchangeably herein. Examples include anti-skin cancer compositions containing propolis and cyclodextrin or anti-skin cancer compositions containing materials with propolis contained and cyclodextrin. Synergistic compositions which enhance any anti-skin cancer activity observed in either propolis or in cyclodextrin alone are particularly contemplated. The anti-skin cancer compositions may be anti-basal cell carcinoma, anti-squamous cell carcinoma or anti-melanoma compositions.

The term "and/or" can mean "and" or "or".

The terms "cancer" and "cancerous" refer to a physiological condition in mammals that is typically characterized by abnormal or unregulated cell proliferation, cell survival, cell motility, neoplasticity, and/or oncogenicity. Cancer and cancer pathology can be associated, for example, with metastasis, interference with the normal functioning of neighbouring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc. Specifically included are basal cell carcinomas, squamous cell carcinomas, melanomas, and precancerous conditions, which can include dermal tumours, epithelial tumours, tumours of the buccal cavity, for example, squamous cell cancers of the buccal cavity, carcinomas in situ, as well as invasive basal cell, squamous cell, or melanoma cancers, and secondary tumours derived therefrom.

The term "comprising" as used in this specification means "consisting at least in part of". When interpreting statements in this specification that include that term, the features, prefaced by that term in each statement, all need to be present but other features can also be present. Related terms such as "comprise" and "comprised" are to be interpreted in the same manner.

An "effective amount" is the amount required to confer therapeutic effect. The interrelationship of dosages for animals and humans (based on milligrams per meter squared of body surface) is described by Freireich, et al. (1966). Body surface area can be approximately determined from height and weight of the subject. See, e.g., Scientific Tables, Geigy Pharmaceuticals, Ardley, New York, 1970, 537. Effective doses also vary, as recognized by those skilled in the art, dependent on route of administration, excipient usage, and the like.

As used herein, an "extract" or a "fraction" of propolis is suitable for use in the present invention provided they at least retain one or more anti-skin cancer activity exhibited by propolis. Such functional extracts or functional fractions may have greater or lesser activity than the crude propolis. In one example, one or more of the biological activities of the crude propolis possessed by the functional extract or functional fraction may be present to a greater or lesser degree in the functional extract or functional fraction than is found in the crude propolis. In another example, each of the biological activities of the crude propolis possessed by the functional extract or functional fraction is present to a greater or lesser degree in the functional extract or functional fraction than is found in the crude propolis. In still a further example, it may be desirable to provide a functional extract or functional fraction in which one or more of the biological activities of the crude propolis is maintained or is present to a greater degree than is found in the crude propolis, but one or more other biological activities of the crude propolis is not present or is present to a lesser degree than is found in the crude propolis. Examples of such functional extracts include the anti-skin cancer tincture described herein in the Examples.

Methods and assays to determine one or more biological effects elicited by propolis are well known in the art and examples are described herein, and such methods and assays can be used to identify or verify one or more functional extracts or functional fractions of propolis. For example, an assay of the ability of propolis to increase one or more oncogenic traits in a cell, such as those described herein in the Examples, is amenable to identifying one or more functional extracts or functional fractions of propolis.

As used herein, "propolis" contemplates propolis produced by bees from any botantical source. In one embodiment, the propolis is "European" propolis. "European" propolis is also known under different names, such as "Poplar" propolis. For example, the propolis is derived principally from the bud and leaf exudates of one or more species of poplars, and to a lesser extent birches, larches or willows. Propolis has been classified into seven major classes based on plant source which gives rise to characteristic compounds being present (Sforcin and Bankova, 2011. Propolis: is there a potential for the development of new drugs? J. Ethnopharmacology, 133: 253-260.). These classes are "Poplar" from Europe, North America, Southern South America, New Zealand which have high levels of aglycone flavonoids such as chrysin and galangin; "Brazilian green", which contains prenylated p-coumaric acids such as artepillan C; "Birch" from Russia also rich in aglycone flavonoids such as apigenin, rhamnocitrin and kaemferide; "Red propolis" sourced from *Clusia* species from Cuba, Brazil, Mexico Venezuala which contains polyprenylated benzophenones including nemorosone and xanthochymol; "Mediterranean" from Greece, Sicily, Crete, Malta rich in diterpenes that are sourced from conifers; and "Pacific" from Okinawa, Taiwan, Indonesia, which contain 'propolins'.

Exemplary compounds and concentrations of the phenolic compounds reported in European type propolis from various countries are presented in Table 1 below (adapted from Kumazawa et al., 2004 Identification of metabolites in plasma and urine of Uruguayan propolis-treated rats. Journal of Agricultural and Food Chemistry 52(10): 3083-3088.), along with a comparative example of Brazilian green propolis.

**Table 1: Compositional data for ethanol soluble extract of propolis from various geographic regions**

| | **Arg** | **Aus** | **Bra** | **Bul** | **Chil** | **Chi a** | **Chi b** | **Chi c** | **Hun** | **SA** | **Ukr** | **Uru** | **USA** | **Uzb** | **NZ** | **MHNZ1** | **MHNZ2** |
|---|---|---|---|---|---|---|---|---|---|---|---|---|---|---|---|---|---|
| Caffeic acid | 0.7 | 1.7 | 1.6 | 7.2 | 0.4 | 3.3 | 2.8 | 2.4 | 3.1 | 0.2 | 0.8 | 0.7 | 0.8 | 1.5 | 2.8 | 9.4 | 7.2 |
| *p*-Coumaric acid | 1.8 | 3.6 | 27.4 | 3.5 | 1.9 | 4 | 4 | 3 | 3.7 | 1.5 | 8.9 | 8.4 | 19.4 | 0.9 | 3.1 | 3.4 | 2.6 |
| 3,4-Dimethoxy cinnamic acid | 2.2 | 8.6 | | 4 | 1.8 | 10.1 | 7.4 | 7.9 | 5.2 | | 0.8 | 1.1 | 2 | 2.6 | 9.2 | NQbp | NQbp |
| Quercetin | 2.2 | 4.8 | | 4.7 | 1.5 | 4.4 | 3.8 | .8 | 4.4 | | | 2.5 | 3.8 | 0.8 | 1.2 | | |
| Pinobanksin-5-methyl ether | 15 | 23.8 | | 19.7 | 18.8 | 19.8 | 26.2 | 21.1 | 21.8 | 5.9 | 7.5 | 51 | 23.8 | 10.8 | 20 | NObp | NObp |
| Apigenin | 12 | 18.4 | | 13.4 | 14.2 | 17.1 | 17.1 | 14.3 | 9 | | 3.9 | 14.8 | 0.6 | 8.1 | 78.3 | | |
| Kaemferol | 2.3 | 3.9 | | 5 | 1.4 | 2.1 | 2.6 | 2.5 | 4.8 | 1 | 10.9 | 2.5 | 10.3 | 3.8 | 3.7 | | |
| Pinobanksin | 22.5 | 32.1 | | 84.8 | 21.4 | 36.1 | 35 | 22.5 | 21.3 | 31.4 | 6.6 | 36.5 | 23.2 | 29.4 | 36.3 | 35.2 | 25 |
| Cinnamylidene-a¹ | 30.4 | 14.6 | | 6.3 | 31.2 | 11.2 | 12.7 | 10.5 | 7.8 | | | 13.7 | 5.2 | 2.9 | 18.1 | NQbp | NQbp |
| Chrysin | 68.5 | 139 | | 120 | 66.3 | 127.3 | 138 | 138 | 82.9 | 11.2 | 12.9 | 77.3 | 39.4 | 87 | 102 | 32.9 | 24.9 |
| Pinocembrin | 68.7 | 58.7 | | 94.4 | 86.2 | 54.8 | 61.5 | 46.9 | 51.2 | 69.8 | 9.2 | 75 | 46.7 | 44.5 | 100 | 119 | 95.7 |
| Galangin | 32.5 | 42.5 | | 45.6 | 37.7 | 39.6 | 33.5 | 32.6 | 44.2 | 18.9 | 13.4 | 48.8 | 21.5 | 41.4 | 58.2 | 50.6 | 37.3 |
| Pinobanksin-3-acetate | 56.3 | 79.7 | | 41.2 | 63.4 | 52.5 | 64.2 | 51.2 | 59.9 | 7.7 | 14.7 | 80 | 27.6 | 58.4 | 66.2 | 71.3 | 65.5 |
| CAPE | 8.6 | 10.4 | | 5.6 | 7.4 | 29.2 | 24.5 | 19.3 | 15.4 | | 2.6 | 12.4 | 7.2 | 18.6 | 12 | 9.4 | 6.1 |
| Cinnamyl caffeate | 6.6 | 16.6 | | 0.7 | 6.1 | 16.3 | 20.3 | 14.4 | 13.8 | | 2.1 | 8.9 | 7.5 | 2.2 | 12.7 | NQbp | NQbp |
| Tectochrysin | 31.4 | 58.2 | | 96.9 | 33.1 | 62 | 45.4 | 35.5 | 39.0 | 7.7 | 12.4 | 23.8 | 36.1 | 7.3 | 62.2 | 3.5 | 3.3 |
| Artepillin-c | | | 43.9 | | | | | | | | | | | | | | |

| | | | | | | | | | | | | | | | | | |
|---|---|---|---|---|---|---|---|---|---|---|---|---|---|---|---|---|---|
| All values are mg/g propolis resin. NQbp = Not quantified but present ¹Cinnamylideneacetic acid (or phenyl penta-2,4-dienoic acid). Arg = Argentina, Aus = Australia, Bra = Brazilian green? propolis, Bul = Bulgaria, Chil = Chile, Chi = Chinese (a = Hebei, b = Hubei and c = Zheijiang province), Hun = Hungary, NZ = New Zealand, SA = South Africa, Ukr = Ukraine, Uru = Uruguay, Uzb = Uzbekistan, MHNZ 1 = New Zealand propolis (Manuka Health NZ Ltd), MHNZ 2 = New Zealand propolis (Manuka Health NZ Ltd). | | | | | | | | | | | | | | | | | |

These compounds are useful, for example in identifying the source of propolis as being a European-type propolis, or in characterizing and identifying propolis suitable for use in the present invention. It has been reported that Brazilian green propolis contains none of the aglycone flavonoid and caffeic acid ester compounds characteristic of European-type propolis.

As will be appreciated by those skilled in the art, the identity, and in certain cases the suitability for particular uses, including use in the present invention, of propolis may be determined by analysis of the composition of the propolis. The presence and amount of specific compounds, (including the compounds discussed herein) - frequently referred to as "marker compounds", allows a determination of the suitability of a particular source of propolis, for example European propolis compared to Brazilian propolis, for a particular use. In certain embodiments of the present invention, the presence or amount of one or more marker compounds is assayed, as a preliminary grading step, prior to formulation of the composition of the invention.

When used in respect of an agent having anti-skin cancer activity, such as a composition of the invention or a component of a composition of the invention, the phrase "retaining anti-skin cancer activity" and grammatical equivalents and derivatives thereof is intended to mean that the agent still has useful anti-skin cancer activity. For example, the retained activity is at least about 35, 40, 45, 50, 55, 60, 65, 70, 75, 80, 85, 90, 95, 99 or 100% of the original activity, and useful ranges may be selected between any of these values (for example, from about 35 to about 100%, from about 50 to about 100%, from about 60 to about 100%, from about 70 to about 100%, from about 80 to about 100%, and from about 90 to about 100%). Exemplary compositions of the invention are capable of supporting the maintenance of useful anti-skin cancer activity of the anti-skin cancer agent (s) they comprise, and can be said to retain anti-skin cancer activity, ideally until utilized in the methods contemplated herein.

When used in respect of a composition of the invention or a component of a composition of the invention, the phrase "enhancing anti-skin cancer activity" and grammatical equivalents and derivatives thereof is intended to mean that when present in the composition, an equivalent amount or concentration of the anti-skin cancer agent has increased anti-skin cancer activity compared to that of the agent in the absence of the composition (such as the isolated agent). For example, the enhanced activity is at least about 105, 110, 115, 120, 125, 130, 135, 140, 145, 150, 155, 160, 165, 170, 175, 180, 185, 190, 195, 200%, or more of the original activity, and useful ranges may be selected between any of these values (for example, from about 105 to about 200%, from about 120 to about 200%, from about 140 to about 200%, from about 150 to about 200%, from about 180 to about 200%, and from about 190 to about 200%). In certain embodiments, compositions of the invention may exhibit enhanced anti-skin cancer activity, that is, exhibit at least about 105, 110, 115, 120, 125, 130, 135, 140, 145, 150, 155, 160, 165, 170, 175, 180, 185, 190, 195, 200%, or more of the anti-skin cancer activity of propolis alone, or of cyclodextrin alone. Similarly, preferred compositions of the invention are capable of supporting the maintenance of enhanced anti-skin cancer activity, and can be said to retain enhanced anti-skin cancer activity, ideally until utilised using the methods contemplated herein. The enhanced activity (including enhanced maintenance of activity) is believed, without wishing to be bound by any theory, to result from synergy amongst the various components of the compositions of the invention.

As used herein, the term "stable" when used in relation to a composition of the invention means a composition capable of supporting anti-skin cancer activity for, for example, more than two hours, more than three hours, 6 hours, 9 hours, 12 hours, 15 hours, 18 hours, 20 hours, more than one day, for example about two, about three, about four, for example about five, for example about six days, for example a week, two weeks, three weeks, a month, or longer. It will be appreciated that in certain embodiments, stable compositions include those which have anti-skin cancer activity for a period greater than does the propolis or cyclodextrin alone.

The term "oral administration" includes oral, buccal, enteral and intra-gastric administration.

The term "parenteral administration" includes but is not limited to topical (including administration to any dermal, epidermal or mucosal surface), subcutaneous, intravenous, intraperitoneal, intramuscular and intratumoural (including any direct administration to a tumour) administration.

The term "pharmaceutically acceptable carrier" is intended to refer to a carrier including but not limited to an excipient, diluent or auxiliary that can be administered to a subject as a component of a composition of the invention. Preferred carriers do not reduce the activity of the composition and are not toxic when administered in doses sufficient to deliver an effective amount of propolis or extracts thereof, or, when administered, of another anti-skin cancer agent.

The term "(s)" following a noun contemplates the singular or plural form, or both.

The term "subject" is intended to refer to an animal, for example a mammal, such as a mammalian companion animal, or human. Preferred companion animals include cats, dogs and horses. Other mammalian subjects include an agricultural animal, including a horse, a pig, a sheep, a goat, a cow, a deer, or a fowl, or a laboratory animal, including a monkey, a rat, or a mouse.

The term "treat" and its derivatives should be interpreted in their broadest possible context. The term should not be taken to imply that a subject is treated until total recovery. Accordingly, "treat" broadly includes maintaining a subject's disease progression or symptoms at a substantially static level, increasing a subject's rate of recovery, amelioration and/or prevention of the onset of the symptoms or severity of a particular condition, or extending a patient's quality of life. The term "treat" also broadly includes the maintenance of good health for sensitive individuals and building stamina for disease prevention.

### Exemplary uses of the invention

The compositions of the invention for use in treating or preventing skin cancer, as defined in the claims, may be used in the treatment or prevention of melanomas, neoplastic disorders associated with melanomas, and the symptoms of melanoma, melanoma treatment, and associated disorders. Melanoma (also referred to as malignant melanoma) is a neoplastic condition affecting melanocytes.

Melanomas are the least common, but most aggressive and threatening of the skin cancers. Where possible, the preferred treatment is complete surgical removal which can be curative if metastasis has not occurred.

The compositions of the invention for use in treating or preventing skin cancer, as defined in the claims, may be used in the treatment or prevention of basal cell carcinomas, neoplastic disorders associated with basal carcinoma cells, and the symptoms of basal cell carcinoma, basal cell carcinoma treatment, and associated disorders. Basal cell carcinoma is a neoplastic condition affecting the basal cells of the dermis.

Basal cell carcinoma originates from lowest layer of the epidermis. Where possible, the preferred treatment is complete surgical removal which can be curative.

In certain embodiments, the compositions of the invention for use in treating or preventing skin cancer, as defined in the claims, are used in the treatment or prevention of squamous cell carcinomas, neoplastic disorders associated with squamous carcinoma cells, and the symptoms of squamous cell carcinoma, squamous cell carcinoma treatment, and associated disorders.

Squamous cell carcinoma is a neoplastic condition arising in cells of the middle layer of epidermis. Although squamous cell carcinoma is less common that basal cell carcinoma, metastases are more likely and can be fatal if not treated.

In accordance with the claims, the invention provides compositions for inhibiting skin tumour formation, inhibiting skin tumour growth, inhibiting skin tumour metastasis, or treating or preventing a skin cancer in a subject in need thereof. Without wishing to be bound by any theory, applicants believe that inhibition occurs at least in part, for example, by prevention of UV-initiated damage to DNA or the prevention or reduction in formation of reactive oxygen species.

In certain embodiments, the invention provides compositions, as defined in the claims, thatat least partially reverse the resistance of a neoplastic cell in a subject suffering from a skin cancer to a skin cancer therapy, or to a composition for reversing, wholly or in part, the resistance of a skin cancer-burdened patient to a skin cancer therapy, or to a composition for re-sensitising one or more tumours of a skin cancer-burdened patient which are, or are predicted to either be or become, resistant to treatment with a skin cancer therapy, said compositions comprising, consisting essentially or, or consisting of propolis and cyclodextrin. The one or more tumours may be or may be predicted to be or to become resistant to a skin cancer therapy due increased activation of one or more pro-cancer cell survival signaling pathways within the one or more tumours or within the patient, including increased activation of one or more of the AKT, JNK or JAK/STAT signaling pathways, for example within a sample from the patient, such as a tissue sample, a tumour biopsy, or a blood or plasma sample.

Pro-cancer cell survival signaling pathways implicated in the onset and development of skin cancers are known in the art.

The compositions of the invention may be used in the treatment or prevention of melanomas, neoplastic disorders associated with melanoma cells, and the symptoms of melanoma, melanoma treatment, and associated disorders.

The most common therapies are surgical excision of tumours and radiation therapy. Chemotherapeutic agents may be used in combination surgery and/or radiation.

The compositions of the invention, as defined in the claims, may also be used for maintaining or improving skin health.

This includes the treatment or prevention of a condition associated with poor skin health, low immunity and skin inflammation. For example, the compositions of the invention are useful for or in the treatment or prevention of skin aging, sun burn, dermatitis, eczema, psoriasis, ichthyosis and related inflammatory conditions, and in the treatment or prevention of red, irritated, dry, cracked or itchy skin.

### Propolis and materials comprising propolis

Propolis is available in New Zealand and elsewhere, commonly as a resinous sticky solid. Propolis may be obtained from bee-hives with the resulting propolis held in storage, for example to assess the propolis content. Those skilled in the art will recognise that for use in the present invention, propolis may be processed to a form suitable for admixture, for example with cyclodextrin, while maintaining the bioactive ingredients. Typically the propolis, or an extract thereof, is processed to a fine particulate form or a concentrated tincture. Various methods of preparing active propolis, or an extract thereof, to a particulate form or concentrated tincture are known. Most commonly, crude propolis is extracted using ethanol or ethanol/water mixtures to produce a dilute tincture. Wax associated with the crude propolis is at best poorly soluble in the solvent and so is mostly not extracted. Any extracted wax can be removed by cooling the dilute tincture and then settling, filtration, or centrifugation. The tincture can then be concentrated by partial to complete evaporation of the solvent to give a concentrated tincture, optionally followed by freeze drying to give a powder. Alternatively, the tincture can be spray dried to give a powder. Fractions can be prepared by using methods known in the art such as chromatography (such as HPLC) using, for example, a size exclusion matrix or a reverse phase matrix, or supercritical fractionation. A typical solvent for use in such a chromatographic process is ethanol or another water miscible alcohol.

In one embodiment, the composition as defined in the claims comprises propolis or concentrated propolis tincture that is combined with other compounds that enhance the properties of propolis, for example a compound that enhances the ease of formulation or administration, or that enhances anti-skin cancer activity, or that enhances the stability of one or more anti-skin cancer activities present in propolis. Examples of additional compounds are those that improve the therapeutic benefits of the propolis. Exemplary compositions in which one or more compounds present in propolis, and in European propolis in particular, including biologically active compounds such as CAPE, caffeic acid, pinocembrin, benzyl caffeate, cinnamyl caffeate, benzyl ferulate, benzyl caffeate, chrysin, tectochrysin, galangin, pinobanksin, pinostrobin chalcone, and pinobanksin-3-acetate are added are specifically contemplated. In other examples, additional compounds are included to improve or maintain the physiological benefits of the composition, for example mannitol can be added to enhance the diuretic properties of the resulting composition. Alternatively or additionally other compounds such as excipients, and/or propellants could be added to improve the dosing, manufacturability or delivery properties of the composition.

In particularly contemplated embodiments, dewaxed propolis resin, optionally with one or more additional compounds added, is admixed with cyclodextrin and the admixture dried. Further processing of the admixture, for example, to obtain a particle size distribution that enables ready admixture with the other components of the composition, or ease of administration to a subject, is conducted.

In typical embodiments, the propolis or propolis resin is sterilized, for example by heating to kill bacteria, protozoa, yeast, fungi and other organisms that naturally may be present in the propolis.

### Cyclodextrins and materials comprising cyclodextrin

Cyclodextrins are cyclic molecules composed of glucopyranose ring units which form toroidal structures. The interior of the cyclodextrin molecule is hydrophobic and the exterior is hydrophilic, making the cyclodextrin molecule water soluble. The degree of solubility can be altered through substitution of the hydroxyl groups on the exterior of the cyclodextrin. Similarly, the hydrophobicity of the interior can be altered through substitution, though generally the hydrophobic nature of the interior allows accommodation of relatively hydrophobic guests within the cavity. Accommodation of one molecule within another is known as complexation and the resulting product is referred to as an inclusion complex. Cyclodextrins are typically identified with reference to the number of monomeric units that comprise the molecule, wherein alpha-cyclodextrin (α-cyclodextrin) comprises six monomeric units, beta-cyclodextrin (β-cyclodextrin) comprises seven monomeric units, and gamma-cyclodextrin (γ-cyclodextrin) comprises eight monomeric units. Larger cyclodextrin molecules have been described, including a well-characterised cyclodextrin containing 32 1,4-anhydroglucopyranoside units.

Cyclodextrin molecules may conveniently be derivatised, by for example chemical modification, for example to alter one or more of the physicochemical properties thereof. Examples of cyclodextrin derivatives include methylated cyclodextrins, sulfobutylcyclodextrin, maltosylcyclodextrin, hydroxypropylcyclodextrin, for example hydroxypropyl beta-cyclodextrin or hydroxypropyl gamma-cyclodextrin, and salts thereof. Those skilled in the art will recognise that various derivates of cyclodextrin may be suitable for particular purposes, for example, certain derivatives of cyclodextrin are not be acceptable for administration to human subjects, but are suitable for industrial uses.

Cyclodextrins comprising the anti-skin cancer compositions of the present invention may be commercially available, or may be prepared independently by methods well known to those skilled in the art. It will be apparent to those skilled in the art that cyclodextrins used in the anti-skin cancer compositions for administration to a subject, for example a cyclodextrin for manufacturing a beverage, food, or pharmaceutical of the invention should be safe to human body, and for example is a pharmaceutically acceptable cyclodextrin.

In particularly contemplated embodiments compositions of the present invention comprise gamma-cyclodextrin, beta-cyclodextrin or combinations comprising gamma-cyclodextrin and/or beta-cyclodextrin are used. In such embodiments, anti-skin cancer activity is substantially enhanced, as presented herein in the examples. Such compositions comprising gamma-cyclodextrin or beta-cyclodextrin can be formulated to provide, for example, enhanced mouth feel or palatability, for example, compositions comprising gamma-cyclodextrin or beta-cyclodextrin and propolis exhibit a strong tendency to mask any distasteful flavours present in the propolis.

Cyclodextrins suitable for use in the present invention can be obtained from commercial sources, or can be prepared independently by methods well known in the art, such as from starch by enzymatic conversion. In certain embodiments, CAVAMAX W6, W7 or W8 FOOD, an alpha, beta or gamma-cyclodextrin commercially supplied by Wacker AG, is used.

In certain embodiments of the invention, non-food grade cyclodextrins are used, for example, cyclodextrins including substituted cyclodextrins from the CAVASOL range of cyclodextrins are contemplated.

### Compositions of the invention

Exemplary anti-skin cancer compositions of the present invention, in accordance with the claims, include a powder that is obtained after mixing propolis tincture with cyclodextrin, then adding water and homogenizing the composition, and then spray-drying or freeze-drying. Other exemplary anti-skin cancer compositions of the present invention include solutions, including for example, those in which propolis tincture and cyclodextrin are mixed and then dispersed in water, those in which propolis or materials with propolis contained and cyclodextrin are independently dissolved or dispersed in water, and then admixed, for example by kneading, and further those in which propolis powder or resin is firstly dissolved in another organic solvent in which it is soluble, such as for example propylene glycol, ethyl acetate, isopropyl alcohol, and the resultant solution is admixed with cyclodextrin , then added to water, and further mixed, for example by kneading and then dried by means known in the art, such as spray or freeze-drying. In certain embodiments, anti-skin cancer compositions prepared as powders as described above may be preferred, for example because they may maintain stronger anti-skin cancer activity or may maintain anti-skin cancer activity for a longer period than that of solutions of anti-skin cancer compositions prepared as described above.

Without wishing to be bound by any theory, the applicants believe that the propolis in the compositions defined in the claims will be entirely encapsulated when the molar ratio of propolis to cyclodextrin is no greater than 1:1.

In some embodiments the molar ratio of propolis to cyclodextrin may exceed 1:1 in the compositions of the invention. In such compositions the excess propolis will not be encapsulated by the cyclodextrin.

Other anti-skin cancer substances generally known can be combined with the anti-skin cancer compositions of this invention.

Without wishing to be bound by any theory, the applicants believe that the enhanced anti-skin cancer activity observed in exemplary compositions of the present invention may be due at least in part to a synergy between propolis, particularly when present as propolis resin or concentrated fractions of propolis resin, and cyclodextrin. In certain embodiments, the exemplary composition, as defined in the claims, exhibiting a synergy is a composition comprising propolis, particularly a propolis resin or a fraction thereof and gamma-cyclodextrin or beta-cyclodextrin. Again, without wishing to be bound by any theory, the applicants acknowledge that there may be a role of other components of the exemplary compositions, such as polyphenols present in propolis, in achieving the observed enhanced anti-skin cancer activities.

The composition of the invention, as defined in the claims, is formulated for topical administration. The composition of the present invention is for treating or preventing a skin cancer.

In one embodiment, the composition of the invention is in the form of a cream, ointment, a paste, a drop solution including eye drops or ear drops, an aerosol, a dressing, a pad, or a spray.

In one embodiment the composition, as defined in the claims, further comprises one or more constituents (such as antioxidants) which prevent or reduce degradation of the composition during storage or after administration.

Compositions of the invention useful herein may further include other factors such as calcium, zinc, magnesium, selenium, vitamin C, vitamin D, vitamin E, vitamin K2, complex carbohydrates, edible or cooking oils including palm, olive, soybean, canola, corn, sunflower, safflower, peanut, grape seed, sesame, nut, almond, cashew, hazelnut, macadamia, pecan, pistachio, and walnut, and other edibles include acai, amaranth, apricot, argan, artichoke, avocado, babassu, ben, blackcurrant seed, borage seed, borneo tallow nut, bottle gourd, buffalo gourd, carob pod (algaroba), cohune, coriander seed, evening primrose, false flax, hemp, kapok seed, lallemantia, meadowfoam seed, mustard, okra seed (hibiscus seed), perilla seed, pequi, pine nut, poppyseed, prune kernel, pumpkin seed, quinoa, ramtil, rice bran, tea (camellia), thistle, watermelon seed, or wheat germ oil, or a combination thereof.

Those skilled in the art will appreciate that the route of administration to a subject will typically take into account the purpose for which the composition is being administered - for example, where a pharmaceutical composition of the invention is being administered to treat or prevent a skin cancer, the route of administration will typically be chosen taking into account the nature of the health aspect or skin cancer being targeted.

Thus, a pharmaceutical composition useful according to the invention may be formulated with an appropriate pharmaceutically acceptable carrier (including excipients, diluents, auxiliaries, and combinations thereof) selected with regard to the intended route of administration and standard pharmaceutical practice. See for example, Remington's Pharmaceutical Sciences, 16th edition, Osol, A. Ed., Mack Publishing Co., 1980.

Topical formulations comprising compositions of the invention, as defined in the claims, comprising propolis and cyclodextrin, and/or when present the at least one additional anti-skin cancer agent, are particularly contemplated. Topical formulations may be prepared as lotions, creams, ointments, pastes or salves using known carriers for such applications. In certain embodiments, compositions of the invention formulated for topical administration comprise gamma-cyclodextrin, alpha-cyclodextrin or beta-cyclodextrin.

In certain embodiments, topical formulations of the invention comprise one or more penetrants, such as one or more alkyl lactates, one or more antioxidants, such as Vitamin E (alpha-tocopherol) or another naturally-occurring antioxidant including polyphenolic antioxidants such as proanthocyanidins and chlorogenic, quinic, and ferulic acids, one or more photo-protectants or UV-protectants, such as TiO2 or carnosic acid, one or more lipids, collagen, keratin or other proteins.

In certain embodiments, the topical compositions of the invention comprise one or more carriers that are common in cosmetics, such as hydrophilic or lipophilic gelling agents, hydrophilic or lipophilic active agents, preserving agents, antioxidants, solvents, fragrances, fillers, UV-protectants, pigments, odor absorbers and dyestuffs. Typically, the composition will contain an amount conventionally used in the art, for example, from 0.01% to 20% relative to the total weight of the composition. Depending on their nature and the specific embodiment, these carriers are introduced into a lipid phase, into an aqueous phase, or into one or more phases, vesicles, such as one or more lipid vesicles, microparticles, or other components of the topical formulation.

In certain embodiments of the invention, and particularly when the composition of the invention is an emulsion, the proportion of the lipid/fatty phase may range from 5% to 80% by weight, for example from 5% to 50% by weight relative to the total weight of the composition. The oils, emulsifiers and co-emulsifiers contemplated for use in the composition in emulsion form are chosen from those conventionally used in the art. When present, the emulsifier and co-emulsifier are present in the composition in a proportion ranging from 0.3% to 30% by weight, for example from 0.5% to 20% by weight, relative to the total weight of the composition.

In certain embodiments, the composition of the invention comprises one or more oils such as one or more mineral oils, such as liquid petroleum jelly, oils of plant origin, such as avocado oil or soybean oil, oils of animal origin, for example lanolin, synthetic oils, for example perhydrosqualene, silicone oils, such as cyclomethicone, and fluoro oils, including perfluoropolyethers. Fatty alcohols, such as cetyl alcohol, fatty acids and waxes, for example carnauba wax or ozokerite, are also used as fatty substances in certain embodiments of the invention.

In certain embodiments, the emulsifiers and co-emulsifiers are fatty acid esters of polyethylene glycol, such as PEG stearate, or fatty acid esters of glycerol, such as glyceryl stearate, or mixtures thereof.

The use of hydrophilic gelling agents is contemplated in certain formulations of the invention, where such agents include carboxyvinyl polymers, such as carbomer, acrylic copolymers such as acrylate/alkylacrylate copolymers, polyacrylamides, polysaccharides, natural gums and clays, and lipophilic gelling agents including modified clays, for instance bentonites, metal salts of fatty acids, hydrophobic silica and polyethylenes.

Dermabase cream, Unibase cream, and Vanicream are representative examples of commercially available base creams for use as a pharmaceutically acceptable carrier in certain embodiments of the invention.

The topical formulations of the invention will in certain embodiments also contain moisturizers, depigmenting or pigmenting agents, antimicrobial agents, or free-radical scavengers.

In certain embodiments, topical compositions of the invention are formulated as aqueous formulations, such as an aqueous skin cream, for example a water-in-oil or oil-in-water emulsion.

Such formulations may be administered directly, for example, applied directly on to a wound, sprayed onto a surgical site, etc, or may be applied indirectly, such as by impregnation into a bandage or dressing or sprayed onto surgical equipment, dressings and the like.

The efficacy of a composition useful according to the invention, and as defined in the claims, can be evaluated both *in vitro* and *in vivo.* See, e.g., the examples below. Briefly, in one embodiment the composition can be tested for its ability, to for example, inhibit neoplastic cell proliferation *in vitro.* For *in vivo* studies, the composition can be topically applied to an animal (e.g., a mouse) and its effects on skin cancer cell survival, proliferation, metastasis, or one or more symptoms of a skin cancer or associated disease or disorder are then assessed. Based on the results, an appropriate dosage range, frequency, and administration route can be determined.

The compositions useful herein, and as defined in the claims, may be used alone or in combination with one or more other anti-skin cancer agents, or one or more additional therapeutic agents. The anti-skin cancer agent or additional therapeutic agent may be or comprise a food, drink, food additive, drink additive, food component, drink component, dietary supplement, nutritional product, medical food, nutraceutical, cosmeceutical, medical device, medical supply, medicament or pharmaceutical. The anti-skin cancer agent or additional therapeutic agent is for example effective to attenuate one or more neoplastic diseases or disorders or one or more of the symptoms of one or more neoplastic diseases or disorders, or otherwise confer a benefit on the subject to whom it is administered. Preferred therapeutic agents include therapeutic food factors, immunogenic or immunostimulatory agents, wound healing agents, and the like.

It should be understood that the additional anti-skin cancer or therapeutic agents listed above (both food based and pharmaceutical agents) may also be administered separately, simultaneously or sequentially with a composition useful herein.

As will be appreciated, the dose of the composition administered, the period of administration, and the general administration regime may differ between subjects depending on such variables as the severity of symptoms of a subject, the type of disorder to be treated, the mode of administration chosen, and the age, sex and/or general health of a subject. However, by way of general example, from about 1 mg to about 5000 mg per kg body weight of a composition useful herein is administered, 1 mg to about 4000 mg per kg body weight of a composition useful herein is administered, 1 mg to about 3000 mg per kg body weight of a composition useful herein is administered, 1 mg to about 2000 mg per kg body weight of a composition useful herein is administered, 1 mg to about 1000 mg per kg body weight of a composition useful herein is administered, per administration or per day, for example about 50 to about 1000 mg per kg, for example per day. In one embodiment, the administration is of from about 0.05 mg to about 250 mg per kg body weight of a composition useful herein.

In various embodiments, sufficient composition is administered to deliver from about 0.001 mg to about 50 mg of propolis per kg body weight, from about 0.001 mg to about 40 mg of propolis per kg body weight, from about 0.001 mg to about 30 mg of propolis per kg body weight, from about 0.001 mg to about 20 mg of propolis per kg body weight, from about 0.001 mg to about 10mg of propolis per kg body weight, from about 0.001 mg to about 5 mg of propolis per kg body weight, from about 0.001 mg to about 1 mg of propolis per kg body weight, from about 0.001 mg to about 0.5 mg of propolis per kg body weight, from about 0.001 mg to about 0.1 mg of propolis per kg body weight, or from about 0.001 mg to about 0.05 mg of propolis per kg body weight, per administration or per day.

It should be appreciated that administration may include a single dose, such as a single daily dose, or administration of a number of discrete divided doses as may be appropriate. It should be understood that a person of ordinary skill in the art will be able without undue experimentation, having regard to that skill and this disclosure, to determine an effective dosage regime (including dose and timing of administration) for a given condition.

When used in combination with another anti-skin cancer agent or therapeutic agent, the administration of a composition as defined in the claims and the other anti-skin cancer agent or therapeutic agent may be simultaneous or sequential. Simultaneous administration includes the administration of a single dosage form that comprises all components or the administration of separate dosage forms at substantially the same time. Sequential administration includes administration according to different schedules, for example so that there is an overlap in the periods during which the composition useful herein and other therapeutic agent are provided.

Additionally, it is contemplated that a composition, as defined in the claims, may be formulated with additional active ingredients which may be of benefit to a subject in particular instances. For example, therapeutic agents that target the same or different facets of the disease process may be used.

Exemplary anti-skin cancer compositions of the invention, and methods for preparing such compositions will now be described with reference to the following examples.

### EXAMPLES

### Example 1 - Preparation of compositions of the invention

This example describes the preparation and characterization of compositions of the invention comprising propolis and cyclodextrin.
***Complex CD1:*** A propolis - γ cyclodextrin complex containing 21 % by mass propolis solids was made using an ethanolic tincture of propolis containing 40.3 % by weight propolis solids (wax free). 6.35 kg of concentrated propolis tincture was slowly and continuously added to a stainless steel bowl containing 8.13 kg of γ cyclodextrin (CAVAMAX W8, supplied by Wacker AG) whilst stirring with a K-type mixer attached to a planetary mixer. When all the tincture had been added to the cyclodextrin, the resultant paste/powder was mixed for a further ½ hour. The paste was then removed from the bowl, the stirrer changed to a whisk, and then 60.0 kg of water was added to the bowl. Mixing was restarted, and the paste/powder was semi-continuously added. When all the paste/powder was added the solution was then stirred for a further hour. The resultant dispersion was transferred into trays, which were then heated overnight at 40°C to drive off some water and to also induce settling of the encapsulated propolis as a pasty precipitate. The dilute solution was then carefully poured off the top of the trays, the paste was then weighed onto freeze drier trays, and these trays were then placed in a blast freezer. When the tray contents were fully frozen, the trays were transferred to a freeze drier and then freeze-dried to give a yellow, easily crushable powder. The propolis content of the complex was approximately 21 % by mass. The flavonoid and hydroxycinnamic acid content of the complex CD1 is shown in Table 2.
***Complex CD2:*** A propolis - α cyclodextrin complex containing around 22 % by mass NZ propolis solids was made using an ethanolic tincture of propolis containing 40.3 % by weight propolis solids (wax free). 10.91 g of concentrated propolis tincture was mixed in steps by hand in a stainless steel bowl with 12.92 g of α cyclodextrin (CAVAMAX W6, supplied by Wacker AG). When all the tincture had been added to the cyclodextrin, the resultant paste was mixed for a further ½ hour. 96.3 g of water was then added 4 steps by mixing the contents of the stainless steel bowl using a whisk attachment on a lab scale food mixer. When all the water was added the solution was then stirred for a further hour. The bulk of the well mixed solution was then added to a glass round-bottom flask, which was then manually rotated in an acetone-dry ice bath until the flask contents were fully frozen. The flask was then transferred to a lab scale freeze drier and then freeze-dried to give a light yellow, easily crushable powder. The propolis content of the complex was approximately 24 % by mass. The flavonoid and hydroxycinnamic acid content of the complex CD2 is shown in Table 2.
***Complex CD3:*** A propolis - β cyclodextrin complex containing around 22 % by mass NZ propolis solids was made using an ethanolic tincture of propolis containing 40.3 % by weight propolis solids (wax free). 10.06 g of concentrated propolis tincture was mixed in steps by hand in a stainless steel bowl with 12.81 g of β- cyclodextrin (supplied by Sigma Chemicals). When all the tincture had been added to the cyclodextrin, the resultant paste was mixed for a further ½ hour. 98.4 g of water was then added 4 steps by mixing the contents of the stainless steel bowl using a whisk attachment on a lab scale food processor. When all the water was added the solution was then stirred for a further hour. The bulk of the well mixed solution was then added to a glass round-bottom flask, which was then manually rotated in an acetone-dry ice bath until the flask contents were fully frozen. The flask was then transferred to a lab scale freeze drier and then freeze-dried to give a light yellow, easily crushable powder. The propolis content of the complex was approximately 23 % by mass. The flavonoid and hydroxycinnamic acid content of the complex CD3 is shown in Table 2.
***Complex CD4:*** A propolis - γ cyclodextrin complex containing around 20 % by mass Brazilian green propolis solids was made using a propylene glycol solution of propolis containing 40.0 % by weight propolis solids (wax free, supplied by Polenectar, Brazil). 8.13 g of γ cyclodextrin (CAVAMAX W8, supplied by Wacker AG) was dissolved into 60.0 g of water in a stainless steel bowl of a lab scale food processor. When the cyclodextrin was fully dissolved, 6.35 g of the propylene glycol solution of Brazilian green propolis was added drop wise to the solution with stirring using a whisk attachment on the lab scale food processor. When all the propolis solution was added the solution was then stirred for a further ½ hour. The bulk of the well mixed solution was then added to a glass round-bottom flask, which was then manually rotated in an acetone-dry ice bath until the flask contents were fully frozen. The flask was then transferred to a lab scale freeze drier and then freeze-dried to give a light green, easily crushable powder. The Brazilian green propolis - γ cyclodextrin complex contained 3.2 mg/g artepillin-c, 1.2 mg/g para-coumaric acid and 0.1 mg/g caffeic acid. The propolis content was approximately 25 %.
***Complex CD5:*** A propolis - hydroxypropyl-β-cyclodextrin complex containing around 20 % by mass NZ propolis solids was made using an ethanolic tincture of propolis containing 36.0 % by weight propolis solids (wax free). 4.22 g of concentrated propolis tincture was mixed in steps by hand in a mortar using a pestle with 6.01 g of hydroxypropyl-β-cyclodextrin (supplied by Sigma Chemicals). The sticky brown paste was transferred to a beaker with a magnetic flea. 60.1 g of distilled water was added and the mixture stirred for 1 hour. The flea was removed and the mixture was then vigorously stirred with a stick mixer for a further 10 minutes. A portion of the brown/tan coloured opaque solution was transferred to a round bottom flask. The flask contents were then frozen by rotation in a dry ice and acetone bath, before being freeze-dried overnight. 5.9 g of a light yellow dry powder was recovered from the flask. The flavonoid and hydroxycinnamic acid content of the complex is similar to complexes CD1 - 3.
***Complex CD6:*** A propolis resin polyphenol concentrate encapsulated with β-cyclodextrin was made as follows. A propolis tincture containing 10 % dry solids was fractionated by supercritical antisolvent fractionation using the methodology outlined in Catchpole et. al., Journal of Supercritical Fluids, 29, 2004, 97-106 to obtain a first fraction highly enriched in propolis polyphenols (38 % by mass quantified polyphenols including caffeic acid, pinocembrin, pinobanksin, chrysin, galangin, CAPE, pinobanksin-3-O-acetate), and a second fraction with low levels of polyphenols (6 % by mass quantified polyphenols) and high levels of dark coloured compounds. Both fractions were dried by rotary vacuum evaporation to remove any ethanol and water, and then the polyphenol concentration and composition of the fractions was determined by HPLC. A 40 % dry solids tincture was then made by dissolving 2.029 g of the polyphenol-rich fraction in 2.782 g of ethanol and 0.286 g of water. 4.449 g of the tincture was mixed in steps by hand in a mortar using a pestle with 5.994 g of β-cyclodextrin (supplied by Sigma Chemicals). The light yellow paste was transferred to a beaker with a magnetic flea. 60.47 g of distilled water was added and the mixture stirred for 1 hour. The flea was removed and the mixture was then vigorously stirred with a stick mixer for a further 10 minutes. A portion of the almost white coloured opaque solution was transferred to a round bottom flask. The flask contents were then frozen by rotation in a dry ice and acetone bath, before being freeze-dried overnight. 7.11 g of an almost white dry powder was recovered from the flask. The dry cyclodextrin complex contained approximately 23 % propolis solids containing 38 % quantified polyphenols. The flavonoid and hydroxycinnamic acid content of the complex CD6 is shown in Table 2. The levels of the quantified compounds are substantially enhanced relative to the other complexes.
***Complex CD7:*** The dry solids in the second, low polyphenol fraction obtained by supercritical antisolvent fractionation as per Complex CD6 were formulated into a β-cyclodextrin complex containing approximately 26 % propolis dry solids containing 6 % quantified polyphenols. A 41.6 % dry solids tincture was made by dissolving 2.149 g of the polyphenol-poor fraction in 2.739 g of ethanol and 0.276 g of water. 5.114 g of the tincture was mixed in steps by hand in a mortar using a pestle with 6.005 g of β-cydodextrin (supplied by Sigma Chemicals). The dark brown paste was transferred to a beaker with a magnetic flea. 60.9 g of distilled water was added and the mixture stirred for 1 hour. The flea was removed and the mixture was then vigorously stirred with a stick mixer for a further 10 minutes. A portion of the brown coloured opaque solution was transferred to a round bottom flask. The flask contents were then frozen by rotation in a dry ice and acetone bath, before being freeze-dried overnight. 6.58 g of a yellow dry powder was recovered from the flask. The flavonoid and hydroxycinnamic acid content of the complex CD7 is shown in Table 2. The levels of the quantified compounds are substantially reduced relative to the other complexes.
***Complex CD8:*** A propolis - γ cyclodextrin complex containing 27 % by mass propolis solids was made using an ethanolic tincture of propolis containing 23.1 % by weight propolis solids (wax free). 6.16 kg of γ cyclodextrin (CAVAMAX W8, supplied by Wacker AG) was mixed with 9.50 kg of propolis tincture in a stainless steel tank by hand until a homogenous, pasty brown liquid was obtained. 12.3 kg of water was then added in 4 steps and then the resultant mixture was homogenized at 6000 rpm for 1 hour. A further 30.43 kg of water was then added in 4 steps with continuous stirring. This solution was then spray dried to give a fine yellow powder. The flavonoid and hydroxycinnamic acid content of the complex CD8 is shown in Table 2.
***Complex CD9:*** The same procedure for complex CD1 was used to manufacture propolis - γ cyclodextrin complex with added CAPE, but with the following modification. 12 g of solid CAPE (supplied by Sigma Aldrich at > 97 % purity) was mixed with 9.5 kg of propolis containing 23.1 % by weight solids until fully dissolved. The propolis tincture with added CAPE was then mixed with γ cyclodextrin, and then water, and then spray dried as above. The flavonoid and hydroxycinnamic acid content of the complex CD9 is shown in Table 2. This composition comprises twice the concentration of CAPE as complex CD1.

**Table 2: CAPE and flavonoid composition of cyclodextrin complexes, mg/g of complex**

| **Complex** | **Cyclodextrin** | **CAPE** | **pinobanksin** | **pinobanksin-3-acetate** | **pinocembrin** | **chrysin** | **galangin** |
|---|---|---|---|---|---|---|---|
| CD1 | gamma | 1.2 | 5.0 | 9.1 | 17.7 | 5.7 | 8.1 |
| CD2 | alpha | 1.5 | 5.7 | 10.0 | 21.0 | 6.5 | 9.2 |
| CD3 | beta | 1.4 | 5.3 | 9.6 | 20.2 | 6.3 | 8.9 |
| CD6 | beta | 3.9 | 7.1 | 27.2 | 23.2 | 11.6 | 18.5 |
| CD7 | beta | 0.6 | 1.2 | 1.9 | 2.2 | 4.4 | 4.7 |
| CD8 | gamma | 2.0 | 5.2 | 14.6 | 19.5 | 5.4 | 8.1 |
| CD9 | gamma | 4.9 | 5.1 | 14.4 | 19.1 | 5.3 | 7.9 |

### Example 2: Antiproliferative activity of propolis and cyclodextrin-encapsulated propolis against human melanoma

This example presents an assessment of the anti-skin cancer efficacy of representative compositions of the present invention.

This example describes the *in vitro* bioassay used for determining the anti-proliferative activity of test compounds and cyclodextrin-encapsulated propolis complexes against human skin cancer cell lines. The present example tests anti-proliferative efficacy against the human melanoma cell line, A-2058. The cyclodextrin-encapsulated propolis complexes tested are those described in Example 1. The NZ propolis was the same propolis used to manufacture the cyclodextrin complexes CD1-CD3. The Brazilian Green propolis was the same propolis used to manufacture the cyclodextrin complex CD4. The test complexes, positive controls, working solutions and final concentrations used in the bioassay are shown in Table 3. The test samples were dissolved in 15% Ethanol (EtOH)/HBSS to make a working solution, which was then diluted 10 times to give a final concentration in the cells of 50 µg/ml unless indicated otherwise.

**Table 3: Test compounds, complexes, positive controls and final concentrations**

| **Sample number** | **Compound or complex** | **Sample form: solid or solution in EtOH** | **Working solution, 15 % EtOH/HBSS** | **Final conc. in 1.5 % EtOH/HBSS** |
|---|---|---|---|---|
| PC1 | 5-fluorouracil | | 1.95 µg/ml | 0.195 µg/ml |
| PC2 | 5-fluorouracil | | 6.5 µg/ml | 0.65 µg/ml |
| PC3 | 5-fluorouracil | | 19.5 µg/ml | 1.95 µg/ml |
| S#1 | NZ propolis | 62.5 mg/ml | 0.5 mg/ml | 50 µg/ml |
| S#2 | Brazilian green propolis | 62.5 mg/ml | 0.5 mg/ml | 50 µg/ml |
| S#3 | CD1 | solid | 2 mg/ml | 200 µg/ml |
| S#4 | CD2 | solid | 2 mg/ml | 200 µg/ml |
| S#5 | CD3 | solid | 2 mg/ml | 200 µg/ml |
| S#6 | CD4 | solid | 2 mg/ml | 200 µg/ml |

| | | | | |
|---|---|---|---|---|
| Notes: EtOH = ethanol, HBSS = Hank's balanced salt solution, conc. = concentration | | | | |

### General in-vitro skin cancer bioassay:

The three human skin cancer cell lines were:
1) Human Melanoma cell line (A-2058).
2) Human Epidermoid (Squamous) Carcinoma cell line (A-431).
3) Human Basal Cell Carcinoma cell line (TE 354.T).

The cell lines were revived from cryostorage and cultured in the presence of the test and reference samples. An MTT assay was then performed on the cultures to determine the effect of the samples on the cell viability and proliferation. The percentage standard error of the mean was assessed and extreme outliers were removed if the SEM% >15. Preliminary statistical significance was assessed with an independent Student t-test at α ≤ 0.05 (with and without outliers). The methodology was based on the procedures reported by: Ah
n, NG, Campbell JS (1993). Metabolic labeling of mitogen-activated protein kinase in A431 cells demonstrates phosphorylation on serine and threonine residues. Proc Natl Acad Sci. 90: 5143 - 5147.
Galan-Cobo, A et al (2014). Functional inhibition of aquaporin-3 with a gold-based compound induces blockage of cell proliferation. J Cellul. Physiol. 229: 1787 - 1801.
Roomi, MW, Kalinovsky, J et al (2013) Effect of a nutrient mixture on matrix metalloproteinase-9 dimers in various human cancer cell lines. Inter J Oncol. 44: 936 - 942.
Huang, HC, Lin, MK et al (2013). Cardenolides and bufadienolide glycosides from Kalanchoe tubiflora and evaluation of cytotoxicity. Planta Medica. 79: 1362 - 1369.
Tilli, CMLJ, Stavast-Kooy, AJW et al (2003). The garlic-derived organosulfur component ajoene decreases basal cell carcinoma tumor size by inducing apoptosis. Arch Dermatol Res. 295: 117 - 123.

### Experimental Procedures

### Characterisation of the Test System

1. Human melanoma cell line (A-2058, ATCC CRL11147) was obtained from ATCC, Bethesda, MD, USA.
2. Human squamous (epidermoid) carcinoma cell line (A-431, ATCC CRL1555) was obtained from ATCC, Bethesda, MD, USA.
3. Human basal cell carcinoma cell line (TE 354.T, ATCC CRL7762) was obtained from ATCC, Bethesda, MD, USA.
4. Dulbecco's Modified Eagle's Medium (DMEM) was obtained from GIBCO (12100-038). 10% Foetal Bovine Serum (FBS), 100U/ml Penicillin and 100mg/ml Streptomycin were added before use.
5. Penicillin-streptomycin solution, stored at -20°C consisting of 10000 units/ml penicillin and 10mg/ml streptomycin in 0.9% NaCl was obtained from Sigma (#P-0781).
6. Trypsin-EDTA solution consisting of 0.25% Trypsin/EDTA was obtained from Invitrogen (#15400054).
7. Phosphate buffered saline (PBS) was prepared in-house.
8. Hanks Balanced Salt Solution (HBSS), stored at 4°C was obtained from GIBCO (# 14185-052).
9. Foetal Bovine Serum (FBS) stored at -20° C was obtained from GIBCO (# 10091-148).
10. MTT Reagent, 100 mg/vial, was obtained from Sigma (M-2128) and then dissolved in PBS at 10 mg/ml and stored at -20 °C. A 5mg/ml MTT solution was prepared in PBS and stored at 4 °C as the working solution.
11. MTT lysis buffer: 10 % sodium dodecyl sulphate (SDS)/45% Dimethyl Formamide was prepared by dissolving 20 g SDS in 100 ml of double-distilled water (DDW), and then adding 90 ml of Dimethyl Formamide to the solution. The pH was adjusted to 4.7 by glacial acetic acid, and DDW added up to a final volume of 200 ml.
12. 5-Fluorouracil (5-FU) was supplied by Sigma (F-6627). Three working solutions were prepared at 19.5µg/ml. 6.5µg/ml and 1.95µg/ml dissolved in 15% Ethanol/HBSS. Final concentrations were 1.95µg/ml (15µM), 0.65µg/ml (5µM) and 0.195µg/ml (1.5µM).

### Medium Preparation

The culture conditions for the cells were those provided by the supplier of the cells (ATCC). The medium for the propagation of the melanoma cell line is given above in "characterisation of the test system". The medium was prepared following the ATCC instructions and supplemented with penicillin-streptomycin solution (10ml per litre). FBS (at 10%) was also added just before use.

### Culturing of Cells:

1. Each of the cell lines was revived from cryostorage.
2. Following initial propagation using the media described above (Characterisation of Test System, and Medium Preparation), the cell culture was subcultured using trypsin-EDTA. The media was removed and 5ml of the trypsin-EDTA solution added and incubated at 37°C for 5 min or until all the cells had detached. The trypsin was neutralised by the addition of an equal volume of the relevant medium and centrifugation of the suspension at 300g (1200rpm) for 5 min at 4°C.
3. The supernatant was decanted and the cell pellets re-suspended in DMEM that contained FBS (10%), penicillin (100 units/ml), streptomycin (100µg/ml).
4. After reaching confluence, the cells were detached using trypsin-EDTA as described in 2 above and centrifuged.
5. The supernatants were discarded and the cells re-suspended in DMEM and supplements as described in 3 above at varying concentrations of cells per ml. The cell concentration of the A-431 culture was increased from 1 x 10⁴ to 5 x 10⁴ cells/ml, whereas the concentrations of the other two cell lines (A-2058 and TE 354.T), were increased to 2 x 10⁴ cells/ml.
6. For the three cell lines, into each well of 96 well plates, 180µl of the cells or medium was added. The plates were incubated using 5% CO₂/95% air at 37°C for varying time periods to allow the cells to adhere. For the A-431 culture, the pre-incubation time was 3hrs, whereas the pre-incubation times for the other two cell lines (A2058 and TE 354.T) were 24hrs.
7. 20µl of each of the test compounds or 5-FU was added to each well. To the wells labelled 'medium' or 'cells only', 20µl of 15%ETOH/HBSS was added. The number of times each test sample or control was assessed was noted in the Examples tables.
8. The total volume in each well was 200µl.
9. The plates were further incubated at 37°C in 5% CO₂/95% air for 24hr.

### Cell Proliferation Assay

1. On completion of the incubation, 20 µl of MTT working solution (5mg/ml) was added to all wells and incubated for 3-4 hr at 37° C in 5% CO₂/95% air. The plates were monitored every 30-60 minutes and if a few cells showed the presence of crystals then the lysis buffer was added as in Step 2.
2. 100 µl of MTT lysis buffer was then added and the plates incubated overnight at 37°C in 5% CO₂/95% air. The plates were centrifuged at 1200rpm for 10 minutes to pellet any remaining insoluble material. From each well a 200µl aliquot was transferred to fresh 96 well plates. The plates were read in a VersaMax microplate reader at 550 nm to give the Optical Density (OD) per well. An average OD and standard deviation were determined over the number of replicates for each test compound, positive control and for the cell only control.
3. The % inhibition was calculated as the ratio of the average OD sample to average OD cells only control. The blank reading was subtracted from all wells as a background reading.

### Results

The results of the cell proliferation assay for the samples listed in Table 3 are shown in Table 4, along with results for the positive control 5-fluorouracil (5-FU) at three concentrations (PC1-3), and the negative control (NC, cells only with medium and no test compound or positive control). In the Table, OD is Optical Density measured at 570 nm; SEM is the Standard Error associated with the Mean Optical Density value measured; p is the probability value that the measurement is statistically significant via the Student t-test, here taken to be < 0.05; (NS= not significant); % inhibition is the percentage reduction of proliferation compared to the negative control, with a large number indicating the test compound has anticancer potential.

**Table 4: Anti-proliferative activity of propolis and cyclodextrin-encapsulated propolis complexes against human melanoma cell line A-2058**

| **Sample and replicates** | **Compound or complex** | **Mean OD** | **SEM** | **p-value** | **% inhibition** |
|---|---|---|---|---|---|
| NC, n=6 | Cells only | 0.2013 | 0.0034 | 1 | 0 |
| PC1, n=6 | 5-fluorouracil | 0.1937 | 0.0065 | NS | 3.73 |
| PC2, n=6 | 5-fluorouracil, | 0.1832 | 0.0044 | 8.53E-03 | 8.99 |
| PC3, n=6 | 5-fluorouracil | 0.1847 | 0.0058 | 3.28E-02 | 8.25 |
| S#1, n=6 | NZ propolis | 0.1498 | 0.0015 | 6.93E-08 | 25.55 |
| S#2, n=3 | Brazilian green propolis | 0.1772 | 0.0136 | 5.00E-02 | 11.93 |
| S#3, n=6 | CD1 gamma-CD NZ propolis | 0.1595 | 0.0116 | 6.22E-03 | 20.77 |
| S#4, n=3 | CD2 alpha-CD NZ propolis | 0.1316 | 0.0087 | 3.56E-05 | 34.59 |
| S#5, n=3 | CD3 beta-CD NZ propolis | 0.1175 | 0.0093 | 1.39E-05 | 41.63 |
| S#6, n=6 | CD4 gamma-CD Brazilian green propolis | 0.2017 | 0.0097 | NS | 0 |

| | | | | | |
|---|---|---|---|---|---|
| Notes: CD = cyclodextrin, | | | | | |

### Discussion

The 5-fluorouracil (**5-FU**) was used as a positive control, as it is known to be an inhibitor of the melanoma A-2058 cell line. It was a relatively weak inhibitor of the growth of the melanoma cells at both 0.65 µg/ml and 1.95 µg/ml although both effects were statistically significant at these low concentrations. The gamma, alpha and beta-cyclodextrin complexes tested at 200 µg/ml contained 21, 24 and 23 % propolis solids, and so had an effective propolis concentration of 42, 48 and 46 µg/ml respectively. The gamma-cyclodextrin complex of NZ propolis (CD1) had very similar levels of anti-proliferative activity (21 %) to dry NZ propolis solids (S#1, 26 %) on an equivalent propolis solids basis of 50 µg/ml. However, the alpha (CD2) and beta-cyclodextrin complexes (CD3) of NZ propolis had substantially superior anti-proliferative activity to NZ propolis on the same dry propolis solids basis at 35 and 42 % respectively. The gamma-cyclodextrin complex of Brazilian green propolis tested at 200 µg/ml contained 25 % propolis solids, and so had an effective propolis concentration of 50 µg/ml. Brazilian green propolis tested at 50 µg/ml had weak anti-proliferative activity at 12 %, while the equivalent gamma cyclodextrin complex had no statistically significant activity.

### Example 3: Anti-proliferative activity of purified compounds against human melanoma

This example describes an assessment of the anti-skin cancer efficacy of representative compositions of the present invention. This study was performed using proliferation assays in the human melanoma cell line, A-2058. The bioassay test method was the same as described in Example 2. The test compounds, working solutions and final concentrations used in the bioassay are shown in Table 5. The negative control (cells only) and 5-FU positive controls are the same as for Example 2. The compounds tested are found in the NZ propolis used to manufacture the cyclodextrin complexes in Example 1. All compounds tested are either analytical standards, chemically synthesized and shown to be > 95 % pure or isolated from propolis and extensively purified to be> 95 % pure.

**Table 5: Test compounds and final concentrations**

| **Sample number** | **Compound or complex** | **Sample form: solid or solution in EtOH, TGME^{*}** | **Working solution, 15 % EtOH/HBSS** | **Final conc in 1.5 % EtOH/HBSS** |
|---|---|---|---|---|
| S#7 | pinocembrin | 13.33 mg/ml | 2 mg/ml | 200 µg/ml |
| S#8 | 1,1-DMAC | 13.33 mg/ml | 2 mg/ml | 200 µg/ml |
| S#9 | 3M3BC | 1.333 mg/ml | 0.2 mg/ml | 20 µg/ml |
| S#10 | Pinobanksin-3-o-acetate | 13.33 mg/ml | 2 mg/ml | 200 µg/ml |
| S#11 | Benzyl ferulate | 13.33 mg/ml | 2 mg/ml | 200 µg/ml |
| S#12 | Tectochrysin | 13.33 mg/ml^{*} | 2 mg/ml | 200 µg/ml |
| S#13 | CAPE | 13.33 mg/ml | 2 mg/ml | 200 µg/ml |
| S#14 | *p*-coumaric acid | 13.33 mg/ml | 2 mg/ml | 200 µg/ml |
| S#15 | 5-phenyl penta-2,4-dienoic acid | 13.33 mg/ml | 2 mg/ml | 200 µg/ml |

| | | | | |
|---|---|---|---|---|
| Notes: EtOH = ethanol, TGME = triethylene glycol mono-methyl ether, HBSS = Hank's balanced salt solution; 1,1-DMAC = 1,1-dimethylallyl caffeate; 3M3BC = 3-methyl-3-butenyl caffeate; CAPE = caffeic acid phenethyl ester | | | | |

### Results

The results of the anti-proliferation assays for pure compounds are shown in Table 6. OD is Optical Density measured at 570 nm; SEM is the Standard Error associated with the Mean Optical Density value measured; p is the probability value that the measurement is statistically significant via the Student t-test, here taken to be < 0.05 (NS = not significant); % inhibition is the percentage reduction of proliferation compared to the negative control, with a large number indicating the test compound has anticancer potential. Also included in the bioassay are compounds found in poplar-type propolis with known or expected activity including CAPE (expected to be active) and para-coumaric acid (expected to be inactive). The results for the cells only (negative control), and for the 5-FU positive controls are the same as for Example 2 and so are not included in the Table.

**Table 6: Anti-proliferative activity of propolis pure compounds and complexes against human melanoma cell line A2058**

| **Sample and replicates** | **Compound or complex** | **Mean OD** | **SEM** | **p-value** | **% inhibition** |
|---|---|---|---|---|---|
| S#7, n=6 | pinocembrin | 0.0013 | 0.0011 | 5.92E-14 | 99.35 |
| S#8, n=6 | 1,1-DMAC | 0.0533 | 0.0182 | 5.40E-06 | 73.52 |
| S#9, n=6 | 3M3BC | 0.1070 | 0.0103 | 5.71E-06 | 46.85 |
| S#10, n=6 | Pinobanksin-3-o-acetate | 0.0777 | 0.0044 | 7.58E-10 | 61.38 |
| S#11, n=6 | Benzyl ferulate | 0.0040 | 0.0026 | 4.92E-13 | 98.00 |
| S#12, n=6 | Tectochrysin | 0.0272 | 0.0114 | 4.56E-08 | 86.48 |
| S#13, n=3 | CAPE | 0.1018 | 0.0124 | 1.55E-05 | 49.43 |
| S#14, n=3 | *p*-coumaric acid | 0.2136 | 0.0102 | NS | 0 |
| S#15, n=3 | 5-phenyl penta-2,4-dienoic acid | 0.1548 | 0.0086 | 4.34E-04 | 23.10 |

| | | | | | |
|---|---|---|---|---|---|
| Notes: 1,1-DMAC = 1,1-dimethlallylcaffeic acid; 3M3BC = 3-methyl-3-butenyl caffeate; CAPE = caffeic acid phenethyl ester | | | | | |

### Discussion

This example shows that a number of compounds present in NZ propolis have anti-skin cancer activity. The most potent of these compounds are pinocembrin and benzyl ferulate, followed by tectochrysin, 1,1-dimethylallyl caffeate, pinobanksin-3-o-acetate and 3-methyl-3-butenyl caffeate. Pinocembrin and benzyl ferulate completely killed all the melanoma cells at a concentration of 200 µg/ml. Tectochrysin was also a very powerful inhibitor of proliferation at 86.5 %, as was 1,1-dimethylallyl caffeateat 73.5 % and pinobanksin-3-o-actetate at 61.4 %. 3-methyl-3-butenyl caffeate was also a strong inhibitor of proliferation at 46.8 % given that its concentration was only 20 µg/ml. CAPE at a concentration of 200 µg/ml was a moderate to strong inhibitor of proliferation at 49.4 %. 5-phenylpenta-2,4-dienoic acid at a concentration of 200 µg/ml was a moderate inhibitor of proliferation at 23.1 %. p-coumaric acid was inactive.

### Example 4: Anti-proliferative activity of propolis and cyclodextrin-encapsulated propolis against human epidermoid cancer

This example describes an assessment of the anti-skin cancer efficacy of representative compositions of the present invention.

This study was performed using proliferation assays in the human epidermoid cancer cell line, A-431. The cyclodextrin-encapsulated propolis complexes tested are those described in Example 1. The NZ propolis was the same propolis used to manufacture the cyclodextrin complex CD5. The test complexes, positive controls, working solutions and final concentrations used in the bioassay are shown in Table 7.

**Table 7: Test compounds, complexes, positive controls and final concentrations**

| **Sample number** | **Compound or complex** | **Sample form: solid or solution in EtOH** | **Working solution, 15 % EtOH/HBSS** | **Final conc. in 1.5% EtOH/HBSS** |
|---|---|---|---|---|
| PC1 | 5-fluorouracil | | 1.95 µg/ml | 0.195 µg/ml |
| PC2 | 5-fluorouracil | | 6.5 µg/ml | 0.65 µg/ml |
| PC3 | 5-fluorouracil | | 19.5 µg/ml | 1.95 µg/ml |
| S#1 | NZ propolis | 62.5 mg/ml | 0.5 mg/ml | 50 µg/ml |
| S#2 | Propolis polyphenol concentrate | 62.5 mg/ml | 0.5 mg/ml | 50 µg/ml |
| S#3 | CD3 β-CD NZ propolis | solid | 2 mg/ml | 200 µg/ml |
| S#4 | CD5 hydroxypropyl-β-CD NZ propolis | solid | 2 mg/ml | 200 µg/ml |
| S#5 | CD6 β-CD polyphenol concentrate | solid | 2 mg/ml | 200 µg/ml |

| | | | | |
|---|---|---|---|---|
| Notes: EtOH = ethanol, HBSS = Hank's balanced salt solution, conc. = concentration | | | | |

### Results

The results of the cell proliferation assay for the samples listed in Table 7 are shown in Table 8, along with results for the positive control 5-fluorouracil (5-FU) at three concentrations (PC1-3), and the negative control (cells only with medium and no test compound or positive control). In the Table, OD is Optical Density measured at 570 nm; SEM is the Standard Error associated with the Mean Optical Density value measured; p is the probability value that the measurement is statistically significant via the Student t-test, here taken to be < 0.05 (NS = not significant); % inhibition is the percentage reduction of proliferation compared to the negative control, with a large number indicating the test compound has anticancer potential.

**Table 8: Anti-proliferative activity of propolis and cyclodextrin-encapsulated propolis complexes against human epidermoid cancer cell line A-431**

| **Sample and replicates** | **Compound or complex** | **Mean OD** | **SEM** | **p-value** | **% inhibition** |
|---|---|---|---|---|---|
| NC, n=6 | Cells only | 0.2621 | 0.0158 | 1 | 0 |
| PC1, 0.195 µg/ml, n=6 | 5-fluorouracil | 0.2364 | 0.0064 | NS | 9.79 |
| PC2, 0.65 µg/ml, n=6 | 5-fluorouracil | 0.2416 | 0.0111 | NS | 7.80 |
| PC3, 1.95 µg/ml, n=6 | 5-fluorouracil | 0.2561 | 0.0086 | NS | 2.26 |
| S#1, 50 µg/ml, n=6 | NZ propolis | 0.1498 | 0.0020 | 1.8E-03 | 42.8 |
| S#2, 50 µg/ml, n=3 | Propolis polyphenol concentrate | 0.1586 | 0.0054 | 3.1E-03 | 39.5 |
| S#3, 200 µg/ml, n=6 | CD3 β-CD NZ propolis | 0.1656 | 0.0118 | 5.4E-03 | 36.8 |
| S#4, 200 µg/ml, n=3 | CD5 hydroxypropyl-β-CD NZ propolis | 0.1702 | 0.0019 | 5.4E-03 | 35.0 |
| S#5, 200 µg/ml, n=3 | CD6 β-CD polyphenol concentrate | 0.1456 | 0.0059 | 1.6E-03 | 44.4 |

| | | | | | |
|---|---|---|---|---|---|
| Notes: CD = cyclodextrin | | | | | |

### Discussion

The 5-fluorouracil (5-FU) was used as a positive control, as it is expected to be an inhibitor of the human epidermoid cancer cell line A-431. It was a weak and statistically insignificant inhibitor of the growth of the epidermoid cancer cells at all test concentrations.

The beta-cyclodextrin (CD3) and hydroxypropyl beta-cyclodextrin propolis complexes (CD5) tested at 200 µg/ml contained 23 and 20 % propolis solids, and so had an effective propolis concentration of 46 and 40 µg/ml respectively. The β-cyclodextrin propolis polyphenol concentrate complex contained 23 % solids, and the β-cyclodextrin propolis low polyphenols complex contained 26 % solids. The β-cyclodextrin complex of NZ propolis (CD3) and the hydroxypropyl β-cyclodextrin complex of NZ propolis (CD5) had very similar levels of anti-proliferative activity (36.8 and 35.0 %) to dry NZ propolis solids (S#1, 42.8 %) on an equivalent propolis solids basis of 50 µg/ml.

The β-cyclodextrin complexes of the propolis polyphenol concentrate (CD6) had substantially similar anti-proliferative activity to the propolis polyphenol concentrate alone on the same dry propolis solids basis at 44.4 and 39.5 % respectively.

### Example 5: Antiproliferative activity of pure compounds from propolis against human epidermoid cancer

This example describes an assessment of the anti-skin cancer efficacy of representative compositions of the present invention. This study was performed using proliferation assays in the human epidermoid cancer cell line, A431. The bioassay test method was the same as described in Example 2. The test compounds, working solutions and final concentrations used in the bioassay are shown in Table 9. The negative control (cells only) and 5-FU positive controls are the same as for Example 4 and so are not included in the Table. The compounds tested are constituents of the NZ propolis used to manufacture the cyclodextrin complexes in Example 1. All compounds tested are either analytical standards, chemically synthesized and shown to be > 95 % pure or isolated from propolis and extensively purified to be> 95 % pure.

**Table 9: Test compounds and final concentrations**

| **Sample number** | **Compound or complex** | **Sample form: solid or solution in EtOH** | **Working solution, 15 % EtOH/HBSS** | **Final conc in 1.5% EtOH/HBSS** |
|---|---|---|---|---|
| S#4 | 1,1-DMAC | 13.33 mg/ml | 2 mg/ml | 200 µg/ml |
| S#5 | 3M3BC | 13.33 mg/ml | 2 mg/ml | 200 µg/ml |
| S#6 | Pinobanksin-3-o-acetate | 13.33 mg/ml | 2 mg/ml | 200 µg/ml |

| | | | | |
|---|---|---|---|---|
| Notes: EtOH = ethanol, HBSS = Hank's balanced salt solution; 1,1-DMAC = 1,1-dimethlallyl caffeate; 3M3BC = 3-methyl-3-butenyl caffeate; | | | | |

### Results

The results of the anti-proliferation assays for pure compounds described herein are shown in Table 10. In the Table, OD is Optical Density measured at 570 nm; SEM is the Standard Error associated with the Mean Optical Density value measured; p is the probability value that the measurement is statistically significant via the Student t-test, here taken to be < 0.05 (NS = not significant); % inhibition is the percentage reduction of proliferation compared to the negative control, with a large number indicating the test compound has anticancer potential. The results for the cells only (negative control), and for the 5-FU positive controls are the same as for Example 4 and so are not included in the Table.

**Table 10: Anti-proliferative activity of propolis pure compounds against human epidermoid cancer cell line A-2058**

| **Sample and replicates** | **Compound or complex** | **Mean OD** | **SEM** | **p-value** | **% inhibition** |
|---|---|---|---|---|---|
| S#8, 200 µg/ml, n=3 | 1,1-DMAC | 0.0000 | 0.0000 | 9.9E-05 | 100.00 |
| S#9, 200 µg/ml, n=3 | 3M3BC | 0.0475 | 0.0118 | 3.3E-04 | 81.88 |
| S#10, 200 µg/ml, n=3 | Pinobanksin-3-o-acetate | 0.0253 | 0.0035 | 1.9E-05 | 90.36 |

| | | | | | |
|---|---|---|---|---|---|
| Notes: 1,1-DMAC = 1,1-dimethlallyl caffeate; 3M3BC = 3-methyl-3-butenyl caffeate | | | | | |

### Discussion

This example shows that a number of compounds present in NZ propolis have novel anti-skin cancer activity. The most potent of these compounds was 1,1-dimethylallyl caffeate, followed by pinobanksin-3-o-acetate and 3-methyl-3-butenyl caffeate. 1,1-dimethylallyl caffeate completely killed all the epidermoid cancer cells at a concentration of 200 µg/ml. Pinobanksin-3-o-acetate and 3-methyl-3-butenyl caffeate were also very strong inhibitors of proliferation and were most likely cytotoxic at the concentration tested.

### Example 6: Antiproliferative activity of pure compounds from propolis against human basal cancer

This example describes an assessment of the anti-skin cancer efficacy of representative compositions of the present invention. This study was performed using proliferation assays in the human basal carcinoma cell line, TE 354.T. The test method was the same as described in Example 2. The test complexes, positive controls, working solutions and final concentrations used in the bioassay are shown in Table 11. The compounds tested are constituents of the NZ propolis used to manufacture the cyclodextrin complexes in Example 1. All compounds tested are either analytical standards, chemically synthesized and shown to be > 95 % pure or isolated from propolis and extensively purified to be> 95 % pure.

**Table 11: Test compounds and final concentrations**

| **Sample number** | **Compound or complex** | **Sample form: solid or solution in EtOH** | **Working solution, 15 % EtOH/HBSS** | **Final conc in 1.5% EtOH/HBSS** |
|---|---|---|---|---|
| PC1, n=6 | 5-fluorouracil | | 1.95 µg/ml | 0.195 µg/ml |
| PC2, n=6 | 5-fluorouracil | | 6.5 µg/ml | 0.65 µg/ml |
| PC3, n=6 | 5-fluorouracil | | 19.5 µg/ml | 1.95 µg/ml |
| S#4 | 1,1-DMAC | 13.33 mg/ml | 2 mg/ml | 200 µg/ml |
| S#5 | 3M3BC | 13.33 mg/ml | 2 mg/ml | 200 µg/ml |
| S#6 | Pinobanksin-3-o-acetate | 13.33 mg/ml | 2 mg/ml | 200 µg/ml |

| | | | | |
|---|---|---|---|---|
| Notes: EtOH = ethanol, HBSS = Hank's balanced salt solution; 1,1-DMAC = 1,1-dimethylallyl caffeate; 3M3BC = 3-methyl-3-butenyl caffeate; | | | | |

### Results

The results of the anti-proliferation assays for pure compounds that are the subject of this invention are shown in Table 12. In the Table, OD is Optical Density measured at 570 nm; SEM is the Standard Error associated with the Mean Optical Density value measured; p is the probability value that the measurement is statistically significant via the Student t-test, here taken to be < 0.05 (NS = not significant); % inhibition is the percentage reduction of proliferation compared to the negative control, with a large number indicating the test compound has anticancer potential. The results for the cells only (negative control), and for the 5-FU positive controls are the same as for Example 4 and so are not included in the Table.

**Table 12: Anti-proliferative activity of propolis pure compounds against human basal cancer cell line A-2058**

| **Sample and replicates** | **Compound or complex** | **Mean OD** | **SEM** | **p-value** | **% inhibition** |
|---|---|---|---|---|---|
| NC, n=6 | Cells only | 0.2696 | 0.0096 | 1.00 | 0.00 |
| PC1, 0.195 µg/ml, n=6 | 5-fluorouracil | 0.2548 | 0.0051 | NS | 5.49 |
| PC2, 0.65 µg/ml, n=6 | 5-fluorouracil | 0.2656 | 0.0061 | NS | 1.49 |
| PC3, 1.95 µg/ml, n=6 | 5-fluorouracil | 0.2553 | 0.0070 | NS | 5.31 |
| S#8, 200 µg/ml, n=3 | 1,1-DMAC | 0.0000 | 0.0000 | 2.7E-07 | 100.00 |
| S#9, 200 µg/ml, n=3 | 3M3BC | 0.2245 | 0.0156 | 3.6E-02 | 16.73 |
| S#10, 200 µg/ml, n=3 | Pinobanksin-3-o-acetate | 0.2416 | 0.0063 | NS | 10.38 |

| | | | | | |
|---|---|---|---|---|---|
| Notes: 1,1-DMAC = 1,1-dimethylallyl caffeate; 3M3BC = 3-methyl-3-butenyl caffeate | | | | | |

### Discussion

This example shows that a number of compounds present in NZ propolis have anti-skin cancer activity. The most potent of these compounds was 1,1-dimethylallyl caffeate, followed by 3-methyl-3-butenyl caffeate and pinobanksin-3-o-acetate.

1,1-dimethylallyl caffeate completely killed all the melanoma cells at a concentration of 200 µg/ml. Pinobanksin-3-o-acetate and 3-methyl-3-butenyl caffeate were weak inhibitors of proliferation at 200 µg/ml. This cell line was more resistant to these compounds than melanoma and epidermoid cell lines of Examples 3 and 5.

### Example 7: Anti-inflammatory activity of propolis, propolis fractions and cyclodextrin-encapsulated propolis complexes

The proliferation of cancers can follow an inflammatory pathway. This example describes an assessment of the anti-inflammatory activity of propolis, fractions of propolis produced by preparative chromatography, and cyclodextrin-encapsulated propolis complexes CD8 and CD9 (see Example 1). This study was performed using LPS-activated neutrophils from rat blood to determine the inhibition of the inflammatory mediators cyclooxygenase enzymes COX I and COX II.

### Production of propolis tincture fractions by column chromatography

Fractionation was carried out using a glass column packed with Merck Lichroprep CI8 reversed phase stationary phase (16 x 4 cm) which had been washed with methanol (MeOH) (200 ml) and equilibrated with 20% aqueous ethanol (EtOH) (500 ml). Propolis tincture dry solids (5.446 g) dissolved in EtOH (5 ml) was loaded onto the top of the column using a piston pump. Elution was carried out as a stepped gradient (250 ml) consisting of 20%, 30%, 40%, 50%, 60%, 70%, 80%, and 90% aqueous EtOH, followed by two 100% EtOH steps, then elution with 2-propanol (IPA),ethyl acetate (EtOAc), acetone, and chloroform (CHCl₃). Solvent from the various fractions was removed under vacuum on a rotary evaporator followed by freeze drying overnight. The two 100% EtOH fractions were pooled as were the remaining four non-polar fractions (IPA, EtOAc, acetone, and CHCl₃) for biological assay work due to their relatively low masses.

The fractions and other test samples are shown in Table 13 according to the percentage of ethanol used in the elution step from the column, e.g. 20%, 30%, 40%, 50%, 60%, 70%, 80 % ethanol. The remaining fractions were not tested. The test concentrations were 200 and 50 µg/ml.

**Table 13: Propolis fractionation mass and test sample numbers**

| **Sample #** | **Test Sample ID and test concentrations,** | **Elution solvent** | **Fraction mass (g)** | **% of recovered mass** |
|---|---|---|---|---|
| IFS#1, 20% | Propolis fraction 1 | 20% EtOH | 0.1127 | 2.07 |
| IFS#2, 30% | Propolis fraction 2 | 30% EtOH | 0.0658 | 1.21 |
| IFS#3, 40% | Propolis fraction 3 | 40% EtOH | 0.2408 | 4.42 |
| IFS#4, 50% | Propolis fraction 4 | 50% EtOH | 0.5022 | 9.22 |
| IFS#5, 60% | Propolis fraction 5 | 60% EtOH | 2.2333 | 41.01 |
| IFS#6, 70% | Propolis fraction 6 | 70% EtOH | 0.9843 | 18.07 |
| IFS#7, 80% | Propolis fraction 7 | 80% EtOH | 0.4697 | 8.62 |
| IFS#8 | Propolis tincture dry solids | | | |
| IFS#9 | CD8 complex | | | |
| IFS#10 | CD9 complex | | | |
| IFS#11 | Gamma cyclodextrin | | | |

### Anti-inflammatory assays

Anti-inflammatory assays were carried out as follows. Neutrophils from rat blood were cultured in the presence of the test sample or positive control before being activated into an inflammatory response through the use of lipopolysaccharide (LPS). The incubation period before addition of LPS was 20 minutes. The cell culture supernatants were then tested by ELISA for the presence of the cyclooxygenase COX-1 and COX-2 marker compounds thromboxane TXB₂ and prostaglandin PGE₂, respectively. The positive control was indomethacin (PC1) for the TXB₂ (COX-1) and PGE₂ (COX-2) inflammatory markers, and was tested at 35.8 µg/ml. The test samples were used at 50 µg and 200 µg of solids/ml, and were tested in triplicate. The incubation period for TXB₂ and PGE₂ was 3 hours.

### Results

HPLC analysis was carried out on propolis fractions IFS#1 - IFS#7 to determine the main phenolic compounds. IFS#1 and 2 consisted mainly of the phenolic acids caffeic acid, p-coumaric acid, isoferulic acid and ferulic acid. IFS#3 contained mainly 3,4-dimethoxycinnamic acid. IFS#4 contained mainly cinnamic acid, pinobanksin and 5-phenylpenta-2,4-dienoic acid. IFS#5 contained the main flavonoids in New Zealand propolis including pinocembrin, pinobanksin-3-O-acetate, chrysin, galangin; and the main caffeate esters including benzyl caffeate, 3-methyl-3-butenyl caffeate, 1,1-dimethylallyl caffeate, and caffeic acid phenethyl ester (CAPE). IFS#6 contained low levels of chrysin and galangin, and enhanced levels of pinostrobin chalcone, benzyl ferulate and benzyl isoferulate. IFS#7 contained high levels of tectochrysin, pinocembrin-7-methyl ether and galangin-7-methyl ether (Izalpinin). The anti-inflammatory assay results are shown in Table 14 for all of the test samples.

**Table 14: Anti-inflammatory activity of propolis fractions, propolis and cyclodextrin-encapsulated propolis**

| **Sample #** | **COX (I), 200 µg/ml** | **COX (I), 50 µg/ml** | **COX (II), 200 µg/ml** | **COX (II), 50 µg/ml** |
|---|---|---|---|---|
| PC1, 5.2 µg/ml chloroquine | | | | |
| PC2, 35.8 µg/ml indomethacin | 38.4 | | 40.0 | |
| IFS#1 | S | S | S | S |
| IFS#2 | S | S, NS | S | S |
| IFS#3 | S | S | S | S |
| IFS#4 | S | S | 6.6, NS | 9.3, NS |
| IFS#5 | S | S | S, NS | 0, NS |
| IFS#6 | S | S | S | S |
| IFS#7 | S | S | S | S, NS |
| IFS#8 | S | S | S, NS | 28.7, NS |
| IFS#9 | S | S | S, NS | S, NS |
| IFS#10 | S | S | S | S |
| IFS#11 | 2.3, NS | S, NS | S, NS | 10.1, NS |

| | | | | |
|---|---|---|---|---|
| S = stimulatory, NS = not statistically significant | | | | |

### Discussion

The results of the anti-inflammatory assays show that the propolis, propolis fractions and propolis cyclodextrin complexes had no inhibitory activity towards COX-1 and COX-2 enzymes respectively. In fact, all samples, except the positive control indomethacin, were stimulatory for TXB₂, and similarly all fractions except fraction IFS#4 and indomethacin were stimulatory for PGE₂.

Thus, propolis, the major propolis flavonoids, caffeate and ferulate esters, pinostrobin chalcone or cyclodextrin-encapsulated propolis do not appear to mediate anti-skin cancer activity through a COX or LOX enzyme (arachidonic acid-mediated) pathway.

### INDUSTRIAL APPLICABILITY

Anti-skin cancer compositions of this invention comprising propolis and cyclodextrin can be used in the pharmaceutical and medical fields, for example in medical devices, medical supplies, pharmaceuticals and compositions. Methods of using such compositions, for example in the treatment of skin cancers and symptoms thereof have application in the medical field.

## Claims

1. A composition comprising, consisting essentially of, or consisting of propolis and cyclodextrin for use in treating or preventing skin cancer,
wherein at least some of the cyclodextrin present in the composition is present as an inclusion complex with one or more compounds present in the propolis, and wherein the composition is formulated for topical administration.

2. The composition for use of claim 1 wherein the skin cancer is a melanoma, squamous cell carcinoma or a basal cell carcinoma.

3. The composition for use of claim 1 or 2, wherein the cyclodextrin is alpha-cyclodextrin, beta-cyclodextrin, gamma-cyclodextrin or a chemically-modified cyclodextrin, or the cyclodextrin is present as a combination of cyclodextrins comprising alpha-cyclodextrin, beta-cyclodextrin, gamma-cyclodextrin, or a combination of any two or more thereof.

4. The composition for use of any one of claims 1 to 3, wherein the composition comprises from 1.0%wt to 50 %wt propolis or from 1% to 30%wt propolis resin.

5. The composition for use of any one of claims 1 to 4, wherein the composition comprises two or more of caffeic acid phenylether ester (CAPE), caffeic acid, pinocembrin, benzyl caffeate, benzyl ferulate, cinnamyl caffeate, cinnamyl ferulate, chrysin, galangin, pinostrobin chalcone, and pinobanksin.

6. The composition for use of any one of claims 1 to 4, wherein the composition comprises pinobanksin-3-acetate.

7. The composition for use of any one of claims 1 to 5, wherein the composition has
a) a CAPE concentration of greater than 1 mg/g,
b) a pinocembrin concentration of greater than 10 mg/g,
c) a galangin concentration of greater than 5 mg/g,
d) a chrysin concentration of greater than 5 mg/g,
e) a caffeic acid concentration of greater than 1 mg/g or
f) a pinobanksin concentration of greater than 1 mg/g.

8. The composition for use of any one of claims 1 to 7, wherein the skin cancer is a squamous cell carcinoma or a basal cell carcinoma, and wherein the composition comprises a compound of formula (II): 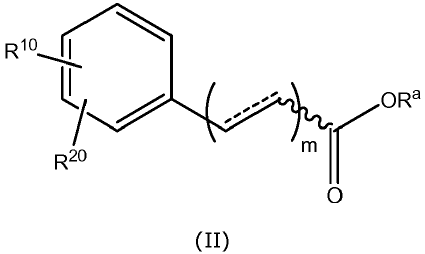 wherein:
R^{a} is hydrogen, C₂₋₆alkenyl, arylC₁₋₆alkyl, or arylC₂₋₆alkenyl;
m is 1 or 2; and
R¹⁰ and R²⁰ are each independently hydrogen, hydroxyl, or C₁₋₆alkoxy; and
provided that R^{a} is not arylC₁₋₆alkyl or arylC₂₋₆alkenyl when R¹⁰ and R²⁰ are both hydroxyl and m is 1;
wherein
preferably the C₂₋₆alkenyl is prenyl or isoprenyl,
preferably the arylC₁₋₆alkyl is benzyl,
preferably the arylC₂₋₆alkenyl is cinnamyl,
preferably the C₁₋₆alkoxy is OMe;
wherein at least some of the compound is present as an inclusion complex with cyclodextrin.

9. The composition for use of claim 8, wherein the compound of formula (II) is a compound of the formula (IIA):

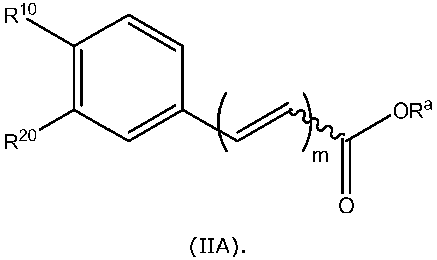

10. The composition for use of claim 8 or 9, wherein R¹⁰ and R²⁰ are each hydrogen, R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl.

11. The composition for use of any one of claims 8 to 10, wherein R^{a} is hydrogen, C₂₋₆alkenyl, or arylC₁₋₆alkyl.

12. The composition for use of any one of claims 8 to 11, wherein m is 1.

13. The composition for use of any one of claims 8 to 12, wherein R^{a} is C₂₋₆alkenyl or arylC₁₋₆alkyl; and R¹⁰ and R²⁰ are each hydroxyl, R¹⁰ is hydroxyl and R²⁰ is C₁₋₆alkoxy, or R¹⁰ is C₁₋₆alkoxy and R²⁰ is hydroxyl.

14. The composition for use of claim 8 or 9, wherein
m is 2, R^{a} is hydrogen, and R¹⁰ and R²⁰ are each independently hydrogen, hydroxyl, or C₁₋₆alkoxy;
m is 1 or 2, R^{a} is C₂₋₆alkenyl, and R¹⁰ and R²⁰ are each independently hydrogen, hydroxyl, or C₁₋₆alkoxy; or
m is 1 or 2, R^{a} is arylC₁₋₆alkyl, R²⁰ is hydrogen, hydroxyl, or C₁₋₆alkoxy, and R¹⁰ is C₁₋₆alkoxy.

15. The composition for use of claim 8, wherein the compound is selected from the group consisting of
a) 1,1-dimethylallyl caffeate,
b) 3-methyl-3-butenyl caffeate,
c) 5-phenylpenta-2,4-dienoic acid, and
d) benzyl isoferulate.

16. The composition for use of any one of claims 1 to 15, wherein the composition is formulated for topical administration to a dermal or epidermal surface of a subject.

## Patentansprüche

1. Zusammensetzung, die Propolis und Cyclodextrin umfasst, im Wesentlichen daraus besteht oder daraus besteht, zur Verwendung bei der Behandlung oder Prävention von Hautkrebs,
wobei zumindest ein Teil des in der Zusammensetzung enthaltenen Cyclodextrins als Einschlusskomplex mit einer oder mehreren in Propolis enthaltenen Verbindungen enthalten ist und wobei die Zusammensetzung zur topischen Verabreichung formuliert ist.

2. Zusammensetzung zur Verwendung nach Anspruch 1, wobei der Hautkrebs ein Melanom, Plattenepithelzellenkarzinom oder ein Basalzellenkarzinom ist.

3. Zusammensetzung zur Verwendung nach Anspruch 1 oder 2, wobei das Cyclodextrin α-Cyclodextrin, β-Cyclodextrin, γ-Cyclodextrin oder ein chemisch modifiziertes Cyclodextrin ist oder das Cyclodextrin als Kombination von Cyclodextrinen vorliegt, die α-Cyclodextrin, β-Cyclodextrin, γ-Cyclodextrin oder eine Kombination zweier oder mehrerer davon umfasst.

4. Zusammensetzung zur Verwendung nach einem der Ansprüche 1 bis 3, wobei die Zusammensetzung 1,0 Gew.-% bis 50 Gew.-% Propolis oder 1 Gew.-% bis 30 Gew.-% Propolisharz umfasst.

5. Zusammensetzung zur Verwendung nach einem der Ansprüche 1 bis 4, wobei die Zusammensetzung zwei oder mehr aus Kaffeesäurephenyletherester (CAPE), Kaffeesäure, Pinocembrin, Benzylcaffeat, Benzylferulat, Cinnamylcaffeat, Cinnamylferulat, Chrysin, Galangin, Pinostrobinchalcon und Pinobanksin umfasst.

6. Zusammensetzung zur Verwendung nach einem der Ansprüche 1 bis 4, wobei die Zusammensetzung Pinobanksin-3-acetat umfasst.

7. Zusammensetzung zur Verwendung nach einem der Ansprüche 1 bis 5, wobei die Zusammensetzung Folgendes aufweist:
a) eine CAPE-Konzentration von mehr als 1 mg/g,
b) eine Pinocembrin-Konzentration von mehr als 10 mg/g,
c) eine Galangin-Konzentration von mehr als 5 mg/g,
d) eine Chrysin-Konzentration von mehr als 5 mg/g,
e) eine Kaffeesäurekonzentration von mehr als 1 mg/g oder
f) eine Pinobanksin-Konzentration von mehr als 1 mg/g.

8. Zusammensetzung nach einem der Ansprüche 1 bis 7, wobei der Hautkrebs ein Plattenepithelzellkarzinom oder ein Basalzellenkarzinom ist und wobei die Zusammensetzung eine Verbindung der Formel (II) umfasst: 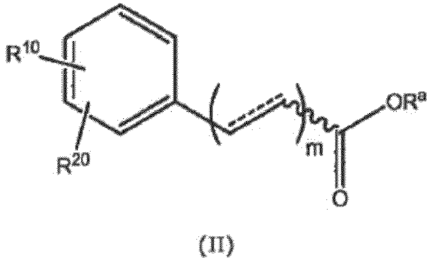 worin:
R^{a} Wasserstoff, C₂₋₆-Alkenyl, Aryl-C₁₋₆-alkyl oder Aryl-C₂₋₆-alkenyl ist;
m = 1 oder 2 ist; und
R¹⁰ und R²⁰ jeweils unabhängig Wasserstoff, Hydroxyl oder C₁₋₆-Alkoxy sind; und
mit der Maßgabe, dass R^{a} nicht Aryl-C₁₋₆-alkyl oder Aryl-C₂₋₆-alkenyl ist, wenn R¹⁰ und R²⁰ beide Hydroxyl sind und m = 1 ist;
wobei
das C₂₋₆-Alkenyl vorzugsweise Prenyl oder Isoprenyl ist,
das Aryl-C₁₋₆-alkyl vorzugsweise Benzyl ist,
das Aryl-C₂₋₆-Alkenyl vorzugsweise Cinnamyl ist,
das C₁₋₆-Alkoxy vorzugsweise OMe ist;
wobei zumindest ein Teil der Verbindung als Einschlusskomplex mit Cyclodextrin vorliegt.

9. Zusammensetzung zur Verwendung nach Anspruch 8, wobei die Verbindung der Formel (II) eine Verbindung der Formel (IIA) ist:

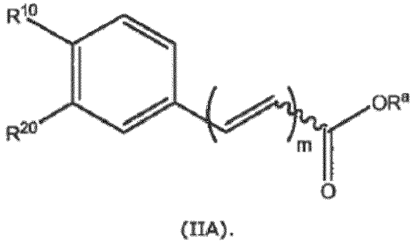

10. Zusammensetzung zur Verwendung nach Anspruch 8 oder 9, wobei R¹⁰ und R²⁰ jeweils Wasserstoff sind, R¹⁰ und R²⁰ jeweils Hydroxyl sind, R¹⁰ Hydroxyl ist und R²⁰ C₁₋₆-Alkoxy ist oder R¹⁰ C₁₋₆-Alkoxy ist und R²⁰ Hydroxyl ist.

11. Zusammensetzung zur Verwendung nach einem der Ansprüche 8 bis 10, wobei R^{a} Wasserstoff, C₂₋₆-Alkenyl oder Aryl-C₁₋₆-alkyl ist.

12. Zusammensetzung zur Verwendung nach einem der Ansprüche 8 bis 11, wobei m = 1 ist.

13. Zusammensetzung zur Verwendung nach einem der Ansprüche 8 bis 12, wobei R^{a} C₂₋₆-Alkenyl oder Aryl-C₁₋₆-alkyl ist; und R¹⁰ und R²⁰ jeweils Hydroxyl sind, R¹⁰ Hydroxyl ist und R²⁰ C₁₋₆-Alkoxy ist oder R¹⁰ C₁₋₆-Alkoxy ist und R²⁰ Hydroxyl ist.

14. Zusammensetzung zur Verwendung nach Anspruch 8 oder 9, wobei
m = 2 ist, R^{a} Wasserstoff ist und R¹⁰ und R²⁰ jeweils unabhängig Wasserstoff, Hydroxyl oder C₁₋₆-Alkoxy sind;
m = 1 oder 2 ist, R^{a} C₂₋₆-Alkenyl ist und R¹⁰ und R²⁰ jeweils unabhängig Wasserstoff, Hydroxyl oder C₁₋₆-Alkoxy sind; oder
m = 1 oder 2 ist, R^{a} Aryl-C₁₋₆-alkyl ist, R²⁰ Wasserstoff, Hydroxyl oder C₁₋₆-Alkoxy ist und R¹⁰ C₁₋₆-Alkoxy ist.

15. Zusammensetzung zur Verwendung nach Anspruch 8, wobei die Verbindung aus der aus Folgenden bestehenden Gruppe ausgewählt ist:
a) 1,1-Dimethylallylcaffeat,
b) 3-Methyl-3-butenylcaffeat,
c) 5-Phenylpenta-2,4-diensäure und
d) Benzylisoferulat.

16. Zusammensetzung zur Verwendung nach einem der Ansprüche 1 bis 15, wobei die Zusammensetzung zur topischen Verabreichung auf eine dermale oder epidermale Oberfläche eines Individuums formuliert ist.

## Revendications

1. Composition comprenant, consistant essentiellement en, ou consistant en de la propolis et de la cyclodextrine pour une utilisation dans le traitement ou la prévention du cancer de la peau,
dans laquelle au moins une partie de la cyclodextrine présente dans la composition est présente sous la forme d'un complexe d'inclusion avec un ou plusieurs composés présents dans la propolis, et dans laquelle la composition est formulée pour une administration topique.

2. Composition à utiliser selon la revendication 1, dans laquelle le cancer de la peau est un mélanome, un carcinome épidermoïde ou un carcinome basocellulaire.

3. Composition à utiliser selon la revendication 1 ou 2, dans laquelle la cyclodextrine est l'alpha-cyclodextrine, la bêta-cyclodextrine, la gamma-cyclodextrine ou une cyclodextrine modifiée chimiquement, ou la cyclodextrine est présente sous la forme d'une combinaison de cyclodextrines comprenant de l'alpha-cyclodextrine, de la bêta-cyclodextrine, de la gamma-cyclodextrine, ou une combinaison de deux de celles-ci ou plus.

4. Composition à utiliser selon l'une quelconque des revendications 1 à 3, dans laquelle la composition comprend de 1,0 % en poids à 50 % en poids de propolis ou de 1 % à 30 % en poids de résine de propolis.

5. Composition à utiliser selon l'une quelconque des revendications 1 à 4, dans laquelle la composition comprend au moins deux ou plus parmi l'ester phényléther d'acide caféique (CAPE), l'acide caféique, la pinocembrine, le cafféate de benzyle, le férulat de benzyle, le cafféate de cinnamyle, le férulat de cinnamyle, la chrysine, la galangine, la pinostrobine chalcone et la pinobanksine.

6. Composition à utiliser selon l'une quelconque des revendications 1 à 4, dans laquelle la composition comprend du pinobanskin-3-acétate.

7. Composition à utiliser selon l'une quelconque des revendications 1 à 5, dans laquelle la composition présente
a) une concentration en CAPE supérieure à 1 mg/g,
b) une concentration en pinocembrine supérieure à 10 mg/g,
c) une concentration en galangine supérieure à 5 mg/g,
d) une concentration en chrysine supérieure à 5 mg/g,
e) une concentration en acide caféique supérieure à 1 mg/g,
f) une concentration en pinobanskine supérieure à 1 mg/g.

8. Composition à utiliser selon l'une quelconque des revendications 1 à 7, dans laquelle le cancer de la peau est un carcinome épidermoïde ou un carcinome basocellulaire, et dans laquelle la composition comprend un composé de formule (II) : 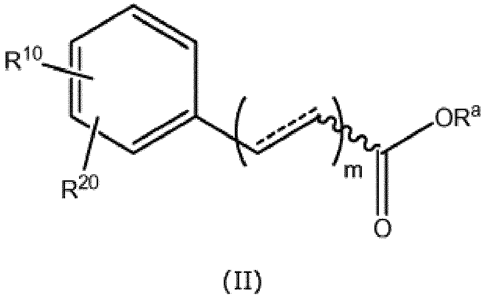 dans laquelle :
R^{a} est de l'hydrogène, un alcényle en C₂₋₆, un aryl-alkyle en C₁₋₆ ou aryl-alécnyle en C₂₋₆ ;
m est 1 ou 2 ; et
R¹⁰ et R²⁰ sont chacun indépendamment de l'hydrogène, un hydroxyle, ou alcoxy en C₁₋₆ ; et à condition que R^{a} ne soit pas un aryl-alkyle en C₁₋₆ ou un aryl-alcényle en C₂₋₆lorsque R¹⁰ et R²⁰ sont tous les deux un hydroxyle et m est 1 ;
dans laquelle :
de préférence l'alcényle en C₂₋₆ est un prényle ou un isoprényle,
de préférence l'aryl-alcényle en C₁₋₆ est un benzyle,
de préférence l'aryl-alcényle en C₂₋₆ est un cinnamyle,
de préférence l'alcoxy en C₁₋₆ est Ome ;
dans laquelle au moins une partie du composé est présente sous forme d'un complexe d'inclusion avec de la cyclodextrine.

9. Composition à utiliser selon la revendication 8, dans laquelle le composé de formule (II) est un composé de la formule (IIA) :

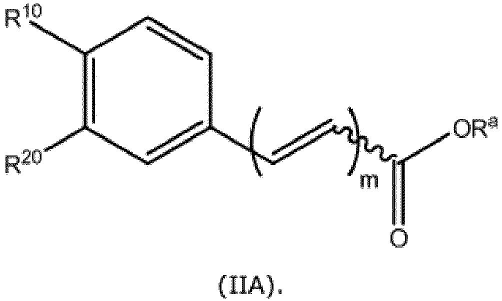

10. Composition à utiliser selon la revendication 8 ou 9, dans laquelle R¹⁰ et R²⁰ sont chacun de l'hydrogène, R¹⁰ et R²⁰ sont chacun un hydroxyle, R¹⁰ est un hydroxyle et R²⁰ est un alcoxy en C₁₋₆ ou R¹⁰ est un alcoxy en C₁₋₆ et R²⁰ est un hydroxyle.

11. Composition à utiliser selon l'une quelconque des revendications 8 à 10, dans laquelle R^{a} est de l'hydrogène, un alcényle en C₂₋₆, un aryl-alkyle en C₁₋₆.

12. Composition à utiliser selon l'une quelconque des revendications 8 à 11, dans laquelle m est 1.

13. Composition à utiliser selon l'une quelconque des renvendications 8 à 12, dans laquelle R^{a} est un alcényle en C₂₋₆ ou un aryl-alkyle en C₁₋₆ ; et R¹⁰ et R²⁰ sont chacun un hydroxyle, R¹⁰ est un hydroxyle et R²⁰ est un alcoxy en C₁₋₆, ou R¹⁰ est un alcoxy en C₁₋₆ et R²⁰ est un hydroxyle.

14. Composition à utiliser selon la revendication 8 ou 9, dans laquelle
m est 2, R^{a} est de l'hydrogène et R¹⁰ et R²⁰ sont chacun indépendamment de l'hydrogène, un hydroxyle ou un alcoxy en C₁₋₆ ;
m est 1 ou 2, R^{a} est de l'hydrogène et R¹⁰ et R²⁰ sont chacun indépendamment de l'hydrogène, un hydroxyle ou un alcoxy en C₁₋₆ ;
m est 1 ou 2, R^{a} est un aryl-alkyle en C₁₋₆, R²⁰ est de l'hydrogène, un hydroxyle ou alcoxy en C₁₋₆, et R¹⁰ est un alcoxy en C₁₋₆.

15. Composition à utiliser selon la revendication 8, dans laquelle le composé est sélectionné dans le groupe comprenant
a) cafféate de 1,1-diméthylallyle,
b) cafféate de 3-méthyl-3-butényle,
c) acide 5-phénylpenta-2,4-diénoïque, et
d) isoférulate de benzyle.

16. Composition à utiliser selon l'une quelconque des revendications 1 à 15, dans laquelle la composition est formulée pour une administration topique à une surface dermique ou épidermique d'un sujet.
